# EUROPEAN PATENT APPLICATION

(11) **EP 4 818 141 A1**
(43) Date of publication of application: **07.10.2026**
(21) Application number: 24897670.6
(22) Date of filing: 28.11.2024
(51) Int. Cl.: C04B 35/488, A61C 5/70, A61C 13/083, A61K 6/818, A61K 6/822, A61K 6/824, A61K 6/831

(54) **ZIRCONIA COMPOSITE SINTERED BODY**

(30) Priority: 28.11.2023 JP 2023201154
(71) Applicant: Kuraray Noritake Dental Inc., Kurashiki-shi, Okayama 710-0801 (JP)
(72) Inventor: NIWA, Takahiro, Miyoshi-shi, Aichi 470-0293 (JP); KATO, Shinichiro, Miyoshi-shi, Aichi 470-0293 (JP)
(74) Representative: D Young & Co LLP
(86) International application number: PCT/JP2024/042238
(87) International publication number: WO 2025/115986

(57) **Abstract**

The present invention provides a zirconia composite sintered body that exhibits excellent aesthetic properties while possessing strength suited for dental use and superior machinability in the sintered state. The present invention relates to a zirconia composite sintered body comprising zirconia, a stabilizer capable of preventing a phase transformation of zirconia, and at least one of Nb₂O₅ or Ta₂O₅, and having a plurality of layers that differ in the content of at least one component from among the stabilizer, Nb₂O₅, and Ta₂O₅ relative to the total moles of zirconia, the stabilizer, Nb₂O₅, and Ta₂O₅.

## Description

### TECHNICAL FIELD

The present invention relates to a zirconia composite sintered body. More specifically, the present invention relates to a zirconia composite sintered body that exhibits excellent aesthetic properties while possessing strength suited for dental use and superior machinability in the sintered state.

### BACKGROUND ART

Ceramics made from metal oxides are used in a wide range of industrial applications. Notably, zirconia sintered bodies have found use in dental materials, such as dental prostheses, due to their high strength and aesthetic qualities.

Because of superior strength, zirconia sintered bodies hardly involve issues such as damage when used in dental materials such as prostheses. Zirconia sintered bodies also have high translucency and resistance to staining in the oral cavity, leading to their superior aesthetic qualities. However, once fully sintered, zirconia sintered bodies exhibit hardness that makes it nearly impossible to process with a dental processing machine. For example, machining a cubic zirconia sintered body into the desired tooth form for a patient results in substantial wear on metal processing tools and demands a considerable amount of time, even for producing just one dental prosthesis.

For these reasons, zirconia sintered bodies, when used in dental material applications, are typically processed into the shape of a desired dental prosthesis while in a more easily processable, semi-sintered state known as a pre-sintered body, rather than as a fully sintered body. The shaped pre-sintered body is then sintered into a sintered body in the required dental prosthesis shape. Afterward, minor adjustments are made to the sintered body to ensure that its shape as a dental prosthesis fits comfortably when placed in the patient's mouth at the dental clinic.

In recent years, CAD/CAM systems have been utilized to machine pre-sintered bodies into the required shape for dental prostheses, allowing customization to fit the patient's teeth at the treatment site. CAD/CAM-compatible pre-sintered bodies (mill blanks) are commonly used for this purpose.

As discussed above, when zirconia sintered bodies are used for dental material applications, major machining after sintering is avoided due to the unique challenges associated with sintering of zirconia. Instead, efforts are directed towards making only minor adjustments to the sintered body when it is placed in the patient's mouth at the dental clinic. In other words, the approach accommodates the stepwise changes in physical properties due to zirconia sintering in dental material applications.

In dental treatment, taking into account the unique circumstances stemming from the physical properties of zirconia sintered bodies, the typical procedure involves a number of steps including: collecting data on the shape within the patient's oral cavity, such as dentition; machining a pre-sintered body (mill blank) into the desired dental prosthesis shape using a CAD/CAM system based on this data; sintering the pre-sintered body in the desired dental prosthesis shape to obtain a sintered body; and making minor adjustments to the sintered body to ensure that it comfortably fits when placed in the patient's mouth at the dental clinic.

Under these circumstances, a pre-sintered body has been proposed that allows for a reduction in the processing time required for firing the aforementioned pre-sintered body to obtain a sintered body (for example, Patent Literature 1).

Patent Literature 1 discloses a zirconia pre-sintered body that comprises zirconia, and a stabilizer capable of preventing a phase transformation of zirconia, wherein the zirconia is predominantly monoclinic zirconia, and the zirconia pre-sintered body has a plurality of layers that differ in stabilizer content relative to the total moles of zirconia and the stabilizer.

However, it is challenging to complete all the steps in a single visit in dental treatment using dental prostheses made of zirconia sintered bodies. As a result, even for the treatment of a single tooth, patients usually need to make multiple trips to the dental clinic, and the entire treatment often takes more than a month to complete.

From the patient's standpoint, it is desirable to minimize the number of clinic visits to reduce the time before the new artificial tooth can be fitted after the treatment is started and to ease the burden of multiple visits. The need for shorter treatment times is growing each year.

If it is possible to perform extensive machining on zirconia sintered bodies in the sintered state, there is no need to machine a pre-sintered body and sinter it to produce a sintered body. Instead, the unprocessed sintered body can be machined into the desired dental prosthesis shape using a CAD/CAM system based on data collected in advance on the oral cavity shape of the patient. This sintered body can then be placed in the patient's mouth and minor adjustments can be made to complete the dental treatment in one day.

Although one-day dental treatments with dental prostheses are possible with non-zirconia materials such as lithium disilicate glass ceramic or feldspathic glass ceramic, accomplishing this with zirconia sintered bodies is highly challenging due to the unique challenges resulting from the physical properties of zirconia sintered bodies.

Given the high demand for zirconia for its strength and aesthetic qualities, and the increasing need for reducing treatment time, there have been proposed zirconia sintered bodies that, unlike conventional zirconia materials involving machining and firing of a pre-sintered body into a sintered body, exhibit superior machinability in the sintered state and can be processed into the desired dental prosthesis shape from prism- or disc-shaped mill blanks (for example, Patent Literatures 2 and 3).

Patent Literature 2 discloses a processable zirconia as a sintered body formed by incorporating a tetragonal zirconia composite powder and a TiO₂ nanopowder, where the tetragonal zirconia composite powder contains 79.8 to 92 mol% ZrO₂ and 4.5 to 10.2 mol% Y₂O₃ along with 3.5 to 7.5 mol% Nb₂O₅ or 5.5 to 10.0 mol% Ta₂O₅, and the TiO₂ nanopowder is incorporated in a mass ratio of more than 0 mass% and 2.5 mass% or less relative to the zirconia composite powder. A method of production of this zirconia sintered body is also disclosed.

Patent Literature 3 discloses a machinable zirconia composite sintered body using raw materials that comprise 78 to 95 mol% ZrO₂ and 2.5 to 10 mol% Y₂O₃, along with 2 to 8 mol% Nb₂O₅ and/or 3 to 10 mol% Ta₂O₅, and in which the primary crystal phase of ZrO₂ is monoclinic. A method of production of this zirconia composite sintered body is also disclosed.

### CITATION LIST

### Patent Literature

Patent Literature 1: WO2020/138316
Patent Literature 2: JP2015-127294 A
Patent Literature 3: WO2021/132644

### SUMMARY OF INVENTION

### Technical Problem

In Patent Literature 1, zirconia powders with different yttria contents are layered to reproduce high transparency layers and opaque layers. However, the need for sintering after machining leads to a longer treatment time for patients, leaving room for improvement in terms of reducing treatment time.

The zirconia sintered bodies disclosed in Patent Literatures 2 and 3 are machinable in the sintered state. However, there is no mention of layering, and the incisal and cervical regions exhibit the same sense of transparency. The present inventors have recognized that further improvement is needed to achieve an appearance more closely resembling natural teeth, even at very close distances, while maintaining machinability in the sintered state.

Another issue is that attempts to improve machinability by lowering material hardness through compositional modification result in reduced material strength. That is, it has been challenging to provide a zirconia sintered body that exhibits excellent machinability in the sintered state while possessing superior strength, and allows for the production of a dental prosthesis that exhibits a sense of transparency that differs between the incisal region and the cervical region and thus more closely resembles natural teeth.

It is an object of the present invention to provide a zirconia composite sintered body that exhibits excellent aesthetic properties while possessing strength suited for dental use and superior machinability in the sintered state.

### Solution to Problem

The present inventors conducted intensive studies to find a solution to the problems discussed above, and found that the foregoing issues can be solved with a zirconia composite sintered body that comprises zirconia, a stabilizer capable of preventing a phase transformation of zirconia, and at least one of Nb₂O₅ or Ta₂O₅, and has a plurality of layers that differ in the content of at least one component from among the stabilizer, Nb₂O₅, and Ta₂O₅ relative to the total moles of zirconia, the stabilizer, Nb₂O₅, and Ta₂O₅. Building upon this finding, the present inventors conducted additional examinations, ultimately resulting in the completion of the present invention.

Specifically, the present invention includes the following.
[1] A zirconia composite sintered body comprising zirconia, a stabilizer capable of preventing a phase transformation of zirconia, and at least one of Nb₂O₅ or Ta₂O₅, and having a plurality of layers that differ in the content of at least one component from among the stabilizer, Nb₂O₅, and Ta₂O₅ relative to the total moles of zirconia, the stabilizer, Nb₂O₅, and Ta₂O₅.
[2] The zirconia composite sintered body according to [1], wherein the plurality of layers are layers that differ in the content of Nb₂O₅ or Ta₂O₅.
[3] The zirconia composite sintered body according to [2], wherein the content of Nb₂O₅ or Ta₂O₅ relative to the total moles of zirconia, the stabilizer, Nb₂O₅, and Ta₂O₅ shows an unchanging pattern of increase or decrease from one end to the other end of the zirconia composite sintered body on a straight line extending along a first direction from one end to the other end of the zirconia composite sintered body.
[4] The zirconia composite sintered body according to [3], wherein the stabilizer is yttria.
[5] The zirconia composite sintered body according to [4], wherein the content of Nb₂O₅ or Ta₂O₅ is 2 mol% or more and 12 mol% or less in a layer including said one end, and the content of Nb₂O₅ or Ta₂O₅ is 1 mol% or more and 10 mol% or less in a layer including said other end, relative to the total moles of zirconia, yttria, Nb₂O₅, and Ta₂O₅.
[6] The zirconia composite sintered body according to [1], wherein the plurality of layers are layers that differ in the content of the stabilizer.
[7] The zirconia composite sintered body according to [6], wherein the content of the stabilizer relative to the total moles of zirconia, the stabilizer, Nb₂O₅, and Ta₂O₅ shows an unchanging pattern of increase or decrease from one end to the other end of the zirconia composite sintered body on a straight line extending along a first direction from one end to the other end of the zirconia composite sintered body.
[8] The zirconia composite sintered body according to [7], wherein the stabilizer is yttria.
[9] The zirconia composite sintered body according to [8], wherein the content of yttria is 1 mol% or more and 8 mol% or less in a layer including said one end, and the content of yttria is 2 mol% or more and 9 mol% or less in a layer including said other end, relative to the total moles of zirconia, yttria, Nb₂O₅, and Ta₂O₅.
[10] The zirconia composite sintered body according to any one of [1] to [9], wherein at least one of the layers in said plurality of layers further comprises an element or ion derived from a capping agent.
[11] The zirconia composite sintered body according to [10], wherein the content of the element or ion derived from a capping agent is more than 0 mol% and 5 mol% or less relative to total 100 mol% of zirconia, the stabilizer, Nb₂O₅, and Ta₂O₅.
[12] The zirconia composite sintered body according to [10] or [11], wherein the element or ion derived from a capping agent belongs to periods 2 to 7 of the periodic table, and is an element or an ion thereof having a first ionization energy lower than that of a group 18 element of the same period, and/or an element or an ion thereof having a high electron affinity.
[13] The zirconia composite sintered body according to [10] or [11], wherein the element or ion derived from a capping agent comprises at least one element or an ion thereof selected from the group consisting of Cu, Ag, Li, Na, K, Rb, Cs, Fr, At, I, Br, Cl, and F.
[14] The zirconia composite sintered body according to [10] or [11], wherein the element or ion derived from a capping agent comprises at least one element or an ion thereof selected from the group consisting of Li, Na, K, Rb, Cs, and Fr.
[15] The zirconia composite sintered body according to any one of [1] to [14], wherein at least one of the layers in said plurality of layers satisfies a ratio A/B of 0.9 or more and 3 or less, where A is the content of the stabilizer in mol%, and B is the combined content of Nb₂O₅ and Ta₂O₅ in mol%.
[16] The zirconia composite sintered body according to any one of [1] to [15], wherein at least one of the layers in said plurality of layers further comprises a zirconia enhancer, and the content of the zirconia enhancer is more than 0 mass% and 6.0 mass% or less relative to total 100 mass% of zirconia, the stabilizer, and Nb₂O₅ and Ta₂O₅.
[17] The zirconia composite sintered body according to [16], wherein the zirconia enhancer comprises TiO₂ and/or Al₂O₃.
[18] The zirconia composite sintered body according to any one of [1] to [17], which has an average crystal grain size of 0.5 to 5.0 µm.

### Advantageous Effects of Invention

According to the present invention, a zirconia composite sintered body can be provided that exhibits excellent aesthetic properties while possessing strength suited for dental use and superior machinability in the sintered state.

Concerning aesthetic properties, the present invention also enables the production of a dental prosthesis that exhibits not only excellent translucency but also a sense of transparency that differs between its incisal region and cervical region after machining and thus more closely resembles natural teeth owning to this gradational translucency, achieving aesthetics comparable to natural teeth even when viewed from a very close distance (for example, about 45 cm).

### DESCRIPTION OF EMBODIMENTS

A zirconia composite sintered body of the present invention comprises zirconia, a stabilizer capable of preventing a phase transformation of zirconia (hereinafter, also referred to simply as "stabilizer"), and at least one of Nb₂O₅ or Ta₂O₅, and has a plurality of layers that differ in the content of at least one component from among the stabilizer, Nb₂O₅, and Ta₂O₅ relative to the total moles of zirconia, the stabilizer, Nb₂O₅, and Ta₂O₅.

As used herein, "molded body" refers to a state in which neither a semi-sintered state (pre-sintered state) nor a sintered state has been reached. That is, "molded body" distinguishes itself from pre-sintered body and sintered body in that it remains unfired after being molded.

As used herein, "zirconia composite pre-sintered body" refers to a semi-sintered state in which a raw material powder such as zirconia has formed necks between particles but has not fully sintered.

As used herein, "zirconia composite sintered body" refers to a sintered state in which a raw material powder such as zirconia has fully sintered. In a zirconia composite sintered body, sintering has caused a raw material powder such as zirconia to consolidate or progressively form a solid solution, increasing the relative density and promoting densification, resulting in a fully sintered state with a relative density of 95% or more.

As used herein, "zirconia" refers to zirconium(IV) oxide (ZrO₂), with a trace amount (0.5 mass% or more and 3 mass% or less) of HfO₂ relative to the amount of ZrO₂. Because HfO₂ is difficult to separate, terms such as "zirconia" and "zirconia powder" are intended to include both ZrO₂ and HfO₂. Likewise, a powder with a stabilizer present in zirconia as a solid solution is also encompassed by "zirconia powder."

As used herein, "in the atmosphere" means under standard atmospheric pressure (1 atm).

As used herein, "zirconia enhancer" refers to a component that serves to enhance the mechanical strength of the zirconia composite sintered body.

In this specification, the content of each component in the zirconia composite sintered body can be calculated from the amounts of raw materials charged.

In this specification, machining encompasses both cutting and grinding. Machining may be a wet or dry process, without specific restrictions.

In this specification, the upper limits and lower limits of numeric ranges (including, for example, temperature ranges, the content of each component, the abundance of crystal systems, components, values calculated from components, and various physical properties) can be appropriately combined.

As used herein, "differing in the content of at least one component from among a stabilizer, Nb₂O₅, and Ta₂O₅" means that the difference in the content of at least one component from among a stabilizer, Nb₂O₅, and Ta₂O₅ between layers is 0.05 mol% or more. The difference is preferably 0.08 mol% or more, more preferably 0.09 mol% or more, even more preferably 0.10 mol% or more, particularly preferably 0.11 mol% or more.

The difference in the content of at least one component from among a stabilizer, Nb₂O₅, and Ta₂O₅ between layers is preferably 3.0 mol% or less, more preferably 2.8 mol% or less, even more preferably 2.5 mol% or less, particularly preferably 2.0 mol% or less.

A zirconia composite sintered body of the present invention has a layered structure in which zirconia, a stabilizer capable of preventing a phase transformation of zirconia, and at least one of Nb₂O₅ or Ta₂O₅ are present in all of the layers, and the layered structure includes a plurality of layers that differ in the content of at least one component from among the stabilizer, Nb₂O₅, and Ta₂O₅ relative to the total moles of zirconia, the stabilizer, Nb₂O₅, and Ta₂O₅.

As used herein, "differing in the content of a component relative to the total moles of zirconia, the stabilizer, Nb₂O₅, and Ta₂O₅" means that the content of a component (at least one component from among the stabilizer, Nb₂O₅, and Ta₂O₅) is different between a given layer and another layer when the total moles of zirconia, the stabilizer, Nb₂O₅, and Ta₂O₅ is calculated separately for these layers.

The reason why a zirconia composite sintered body of the present invention exhibits superior machinability in the sintered state while maintaining strength is unclear. However, by additionally incorporating at least one of Nb₂O₅ or Ta₂O₅ along with conventionally stabilized zirconia, it is considered possible to improve fracture toughness (IF method) while minimizing hardness through coarsening of the microstructure.

The following speculation has been made as to why a zirconia composite sintered body of the present invention can exhibit aesthetic properties resembling those of natural teeth.

In the sintered body comprising at least one of Nb₂O₅ or Ta₂O₅ in addition to the stabilizer, the presence of a plurality of layers differing in the content of at least one component from among the stabilizer, Nb₂O₅, and Ta₂O₅ relative to the total moles of zirconia, the stabilizer, Nb₂O₅, and Ta₂O₅ results in larger crystal grains and higher translucency in layers corresponding to the incisal region than in at least layers corresponding to the cervical region, and, conversely, smaller crystal grains and lower translucency in layers corresponding to the cervical region than in at least the incisal region.

The dental prosthesis, obtained after machining, is therefore able to exhibit translucency comparable to or even greater than that of natural teeth, with a gradation that is sufficient even in comparison with natural teeth.

A zirconia composite sintered body of the present invention can exhibit excellent machinability in the sintered state while possessing superior strength, and allows for the production of a dental prosthesis that more closely resembles natural teeth when (1) the content of at least one of Nb₂O₅ or Ta₂O₅ relative to the stabilizer content, (2) the stabilizer content relative to the Nb₂O₅ content, or (3) the stabilizer content relative to the Ta₂O₅ content is different between any given layers of the layered structure in which zirconia, the stabilizer, and at least one of Nb₂O₅ or Ta₂O₅ are present in all of the layers.

In this specification, descriptions relating to a zirconia composite sintered body of the present invention are applicable to all embodiments, unless otherwise specifically stated.

Additionally, descriptions concerning each component and its content are applicable to all layers of the layered structure, unless otherwise specifically stated.

In a zirconia composite sintered body of the present invention, the zirconia content is preferably 78 to 97.5 mol% in total 100 mol% of zirconia, the stabilizer, Nb₂O₅, and Ta₂O₅. In view of superior translucency and strength, the zirconia content is more preferably 79 mol% or more and 96 mol% or less, even more preferably 80 mol% or more and 94 mol% or less, particularly preferably 81 mol% or more and 93 mol% or less.

In a zirconia composite sintered body of the present invention, examples of the stabilizer capable of preventing a phase transformation of zirconia include oxides such as calcium oxide (CaO), magnesium oxide (MgO), yttrium oxide (Y₂O₃), cerium oxide (CeO₂), scandium oxide (Sc₂O₃), lanthanum oxide (La₂O₃), erbium oxide (Er₂O₃), praseodymium oxide (Pr₂O₃, Pr₆O₁₁), samarium oxide (Sm₂O₃), europium oxide (Eu₂O₃), thulium oxide (Tm₂O₃), gallium oxide (Ga₂O₃), indium oxide (In₂O₃), and ytterbium oxide (Yb₂O₃). In view of achieving superior machinability and strength in the sintered state and providing a prosthesis that exhibits aesthetics more closely resembling that of natural teeth after machining when combined with other components including at least one of Nb₂O₅ or Ta₂O₅, preferred are Y₂O₃ (yttria) and/or CeO₂. The stabilizer may be used alone, or two or more thereof may be used in combination.

In a zirconia composite sintered body of the present invention, the stabilizer content is preferably 1 to 12 mol% in total 100 mol% of zirconia, the stabilizer, Nb₂O₅, and Ta₂O₅. In view of ease of achieving sufficient machinability, the stabilizer content is more preferably 2 mol% or more and 10 mol% or less. In view of even superior translucency and strength, the stabilizer content is even more preferably 3 mol% or more and 8.0 mol% or less, yet more preferably 3.5 mol% or more and 7.5 mol% or less, particularly preferably 3.8 mol% or more and 7.0 mol% or less, most preferably 4.0 mol% or more and 6.5 mol% or less.

In a zirconia composite sintered body of the present invention, the content of Nb₂O₅ or Ta₂O₅ is preferably 1 to 9 mol%, more preferably 1.5 mol% or more and 8.5 mol% or less in total 100 mol% of zirconia, the stabilizer, Nb₂O₅, and Ta₂O₅. In view of providing even superior machinability, the content of Nb₂O₅ or Ta₂O₅ is even more preferably 2.5 mol% or more and 8.0 mol% or less, yet more preferably 2.7 mol% or more and 7.0 mol% or less, particularly preferably 2.8 mol% or more and 6.0 mol% or less, most preferably 3.0 mol% or more and 5.5 mol% or less.

With the content of Nb₂O₅ or Ta₂O₅ falling in these ranges, it is possible to achieve sufficient machinability while reducing the occurrence of defects such as chipping.

The zirconia, stabilizer, Nb₂O₅, and Ta₂O₅ are in the aforementioned content ranges in all of the layers in the layered structure of the zirconia composite sintered body.

The number of layers in the layered structure of a zirconia composite sintered body of the present invention is not particularly limited, and may be 2 to 10, 3 to 6, or 4 to 5.

A certain preferred embodiment is, for example, a zirconia composite sintered body with a layered structure having 3 to 5 layers.

When the number of layers in the layered structure of a zirconia composite sintered body of the present invention is n (preferably, n being an integer of 3 to 10), it is preferable, in view of providing an appearance more closely resembling natural teeth, that the difference in translucency ΔL*(W-B) between a first layer (a layer corresponding to the incisal region) and an nth layer (a layer corresponding to the cervical region) is 1.2 or more, more preferably 1.5 or more. In view of achieving aesthetics comparable to natural teeth even when viewed from a very close distance (for example, about 45 cm), the difference is even more preferably 1.8 or more, particularly preferably 2.0 or more.

In view of providing an appearance more closely resembling natural teeth, the difference in translucency ΔL*(W-B) between a first layer (a layer corresponding to the incisal region) and an nth layer (a layer corresponding to the cervical region) is preferably 6.0 or less, more preferably 5.5 or less, even more preferably 5.0 or less, particularly preferably 4.5 or less.

The method of measurement of translucency ΔL*(W-B) is as described in the EXAMPLES below.

Preferred embodiments of a zirconia composite sintered body of the present invention are described below.

A certain preferred embodiment (hereinafter, this embodiment is also referred to as "first embodiment") is, for example, a zirconia composite sintered body comprising zirconia, a stabilizer capable of preventing a phase transformation of zirconia, and at least one of Nb₂O₅ or Ta₂O₅, and having a plurality of layers that differ in the content of Nb₂O₅ or Ta₂O₅ relative to the total moles of zirconia, the stabilizer, Nb₂O₅, and Ta₂O₅.

In the layered structure of the zirconia composite sintered body according to the first embodiment, the content of Nb₂O₅ or Ta₂O₅ may show a changing pattern of increase or decrease, provided that the dental prosthesis produced exhibits an appearance more closely resembling natural teeth.

In view of providing a dental prosthesis that exhibits gradational translucency and an appearance more closely resembling natural teeth, the difference in the content of Nb₂O₅ or Ta₂O₅ between adjacent layers is 0.08 mol% or more and 3.0 mol% or less in an example of the zirconia composite sintered body according to the first embodiment.

In view of providing a dental prosthesis that exhibits gradational translucency and an appearance more closely resembling natural teeth, the zirconia composite sintered body according to the first embodiment is more preferably one in which the content of Nb₂O₅ or Ta₂O₅ relative to the total moles of zirconia, the stabilizer, Nb₂O₅, and Ta₂O₅ shows an unchanging pattern of increase or decrease from one end to the other end of the zirconia composite sintered body on a straight line extending along a first direction from one end to the other end of the zirconia composite sintered body.

As used herein, the term "one end" can be regarded as the side on the incisal region, and "a layer including one end" as a layer on the incisal-region side. Similarly, the term "other end" can be regarded as the side on the cervical region, and "a layer including the other end" as a layer on the cervical-region side.

In view of achieving not only superior translucency but also a sense of transparency that differs between the incisal and cervical regions of the dental prosthesis after machining and facilitating gradational translucency and an appearance more closely resembling natural teeth, the zirconia composite sintered body according to the first embodiment is more preferably one in which the content of Nb₂O₅ or Ta₂O₅ relative to the total moles of zirconia, the stabilizer, Nb₂O₅, and Ta₂O₅ shows an unchanging pattern of decrease from one end to the other end of the zirconia composite sintered body.

In view of achieving even superior strength and providing even superior machinability in the sintered state as well as providing a dental prosthesis that exhibits gradational translucency and an appearance more closely resembling natural teeth, the zirconia composite sintered body according to the first embodiment is more preferably one in which the content of Nb₂O₅ or Ta₂O₅ relative to the total moles of zirconia, the stabilizer, Nb₂O₅, and Ta₂O₅ is 2 mol% or more and 12 mol% or less in a layer including one end, and 1 mol% or more and 10 mol% or less in a layer including the other end, even more preferably one in which the content of Nb₂O₅ or Ta₂O₅ is 2 mol% or more and 8 mol% or less in a layer including one end, and 1 mol% or more and 6 mol% or less in a layer including the other end, particularly preferably one in which the content of Nb₂O₅ or Ta₂O₅ is 2.5 mol% or more and 6 mol% or less in a layer including one end, and 1.5 mol% or more and 5 mol% or less in a layer including the other end.

With Nb₂O₅ or Ta₂O₅ being present in these ranges in a layer including the other end, the zirconia composite sintered body can have even smaller crystal grains, allowing the translucency of a layer including the other end (a layer corresponding to the cervical region) to decrease and fall within a range that allows the resulting dental prosthesis to exhibit an appearance more closely resembling natural teeth.

Another certain preferred embodiment (hereinafter, this embodiment is also referred to as "second embodiment") is, for example, a zirconia composite sintered body comprising zirconia, a stabilizer capable of preventing a phase transformation of zirconia, and at least one of Nb₂O₅ or Ta₂O₅, and having a plurality of layers that differ in the content of the stabilizer relative to the total moles of zirconia, the stabilizer, Nb₂O₅, and Ta₂O₅.

In the layered structure of the zirconia composite sintered body according to the second embodiment, the stabilizer content may show a changing pattern of increase or decrease, provided that the dental prosthesis produced exhibits an appearance more closely resembling natural teeth.

In view of providing a dental prosthesis that exhibits gradational translucency and an appearance more closely resembling natural teeth, the difference in stabilizer content between adjacent layers is 0.08 mol% or more and 3.0 mol% or less in an example of the zirconia composite sintered body according to the second embodiment.

In view of providing a dental prosthesis that exhibits gradational translucency and an appearance more closely resembling natural teeth, the zirconia composite sintered body according to the second embodiment is more preferably one in which the stabilizer content relative to the total moles of zirconia, the stabilizer, Nb₂O₅, and Ta₂O₅ shows an unchanging pattern of increase or decrease from one end to the other end of the zirconia composite sintered body on a straight line extending along a first direction from one end to the other end of the zirconia composite sintered body.

In view of achieving not only superior translucency but also a sense of transparency that differs between the incisal and cervical regions of the dental prosthesis after machining and facilitating gradational translucency and an appearance more closely resembling natural teeth, the zirconia composite sintered body according to the second embodiment is more preferably one in which the stabilizer content relative to the total moles of zirconia, the stabilizer, Nb₂O₅, and Ta₂O₅ shows an unchanging pattern of increase from one end to the other end of the zirconia composite sintered body.

In view of achieving even superior strength and providing even superior machinability in the sintered state as well as providing a dental prosthesis that exhibits gradational translucency and an appearance more closely resembling natural teeth, the zirconia composite sintered body according to the second embodiment is more preferably one in which the content of the stabilizer (preferably, yttria) relative to the total moles of zirconia, the stabilizer, Nb₂O₅, and Ta₂O₅ is 1 mol% or more and 8 mol% or less in a layer including one end, and 2 mol% or more and 9 mol% or less in a layer including the other end, even more preferably one in which the content of the stabilizer (preferably, yttria) is 2 mol% or more and 8 mol% or less in a layer including one end, and 3 mol% or more and 9 mol% or less in a layer including the other end, particularly preferably one in which the content of the stabilizer (preferably, yttria) is 3 mol% or more and 7.5 mol% or less in a layer including one end, and 4 mol% or more and 8.5 mol% or less in a layer including the other end.

When the stabilizer in a layer including the other end is yttria, increasing the yttria content within the foregoing ranges leads to increased translucency even in conventional zirconia sintered bodies stabilized with yttria. However, even smaller crystal grains can be achieved in the zirconia composite sintered body comprising at least one of Nb₂O₅ or Ta₂O₅, allowing the translucency of a layer including the other end (a layer corresponding to the cervical region) to decrease and fall within a range that allows the resulting dental prosthesis to exhibit an appearance more closely resembling natural teeth.

In any of the embodiments of the present invention, in view of achieving even superior machinability in the sintered state, it is preferable that the zirconia composite sintered body additionally comprise an element or ion derived from a capping agent in at least one of the layers in the layered structure. The element or ion derived from a capping agent may be present in all of the layers.

In this specification, elements or ions derived from a capping agent (hereinafter, also referred to as "capping elements or ions") refer to elements or ions that weaken the strength of the crystal interface (hereinafter, also referred to as "grain boundary") by capping the terminals of the bonding sites of zirconia-based composite oxides within a zirconia composite sintered body constituted of zirconia-based composite oxides (hereinafter, the strength of grain boundaries is also referred to as "grain boundary strength").

The capping agent can at least partially cap the crystal grain boundaries.

The term "capping" refers to elements or ions (capping elements or ions) of interest existing at the crystal grain boundaries by binding to the bonding sites of zirconia-based composite oxides instead of the metal elements.

It is believed that the presence of capping elements or ions in the form of +1 cations or -1 anions at the grain boundaries causes electrostatic repulsion between these capping cations or anions, thereby reducing the grain boundary strength.

The content of zirconia, stabilizer, Nb₂O₅, and Ta₂O₅ in the zirconia composite sintered body can be individually measured using a technique, for example, such as inductively coupled plasma (ICP) emission spectral analysis or X-ray fluorescence analysis.

The content (mol%) of elements or ions derived from a capping agent refers to the proportion external to total 100 mol% of zirconia, the stabilizer, Nb₂O₅, and Ta₂O₅. Accordingly, the content of the elements or ions derived from a capping agent in the zirconia composite sintered body can be calculated by converting the quantity (mass) of the raw material added into mol%.

The content (mass%) of the zirconia enhancer refers to the proportion external to total 100 mass% of zirconia, the stabilizer, Nb₂O₅, and Ta₂O₅. Accordingly, the content of the zirconia enhancer in the zirconia composite sintered body can be calculated from the amount (mass) of raw material charged.

It remains unclear as to why a zirconia composite sintered body of the present invention is machinable in the sintered state owning to its high machinability while possessing translucency as well as strength suited for dental use. However, the following speculation has been made.

When present at the crystal grain boundaries in the zirconia composite sintered body comprising zirconia, a stabilizer capable of preventing a phase transformation of zirconia, and Nb₂O₅ and/or Ta₂O₅, the capping elements or ions appear to reduce the grain boundary strength in the form of +1 cations or -1 anions, facilitating particles to separate, thereby improving grindability and machinability.

In the form of +1 cations or -1 anions, the capping elements or ions bind to the bonding sites of zirconia-based composite oxides at the grain boundaries of the zirconia composite sintered body. This binding leads to electrostatic repulsion between the cations or anions, reducing the grain boundary strength while maintaining the strength and translucency properties of the particles constituting the zirconia composite sintered body, resulting in improved machinability.

As a possible example of such bonding, a +1 cation can form a bond with the other bonding site of an oxygen atom that is already bonded to the metal element (for example, Zr, Hf, Y, Nb, or Ta) contained in the zirconia-based composite oxide, instead of these metal elements forming such bonds.

Another possible example is where a -1 anion binds to the OH₂⁺ attached to the metal element (for example, Zr, Hf, Y, Nb, or Ta) contained in the zirconia-based composite oxide.

In yet another possible example, a -1 anion can bind to the cation derived from the metal element (for example, Zr, Hf, Y, Nb, or Ta) contained within the zirconia-based composite oxide and attached to other metal elements.

In the zirconia composite sintered body, Nb₂O₅ and/or Ta₂O₅ serve to coarsen the microstructure and reduce hardness, and, by acting together with the capping elements or ions, further improve machinability. Through this combined action, Nb₂O₅ and/or Ta₂O₅ and the capping elements or ions can provide even superior free-machinability while ensuring the strength needed as artificial teeth, reducing machining time and increasing the number of dental prostheses that can be produced in continuous processing with a single processing tool while reducing wear on processing tools.

In a zirconia composite sintered body of the present invention, the capping elements or ions further improve machinability, and serve as a free-cutting aid in the manner described above, without greatly compromising strength and translucency.

When at least one of the layers in the plurality of layers constituting a zirconia composite sintered body of the present invention is a layer additionally comprising elements or ions derived from a capping agent, the content of the capping elements or ions contained therein is preferably more than 0 mol% and 5 mol% or less. In view of providing even superior machinability in the sintered state and increasing the number of dental prostheses that can be produced in continuous processing with a single processing tool, the content of capping elements or ions is more preferably 0.05 mol% or more and 3 mol% or less, even more preferably 0.06 mol% or more and 2.5 mol% or less, particularly preferably 0.07 mol% or more and 1.0 mol% or less, most preferably 0.08 mol% or more and 0.34 mol% or less.

When the capping elements or ions contained in a zirconia composite sintered body of the present invention are group 17 elements or ions, the content of capping elements or ions is more preferably 0.2 mol% or more and 5 mol% or less, even more preferably 0.3 mol% or more and 4 mol% or less, particularly preferably 0.4 mol% or more and 3.5 mol% or less, most preferably 0.5 mol% or more and 3.0 mol% or less in view of providing even superior machinability in the sintered state and increasing the number of dental prostheses that can be produced in continuous processing with a single processing tool.

In the layered structure of the zirconia composite sintered body comprising capping elements or ions, the content of capping elements or ions may be the same across all layers or may be different between layers.

As used herein, "differing in the content of capping elements or ions" means that the content of capping elements or ions is different between a given layer and another layer when the proportion of capping elements or ions, external to total 100 mol% of zirconia, the stabilizer, Nb₂O₅, and Ta₂O₅, is calculated separately for these layers.

In this specification, when the content of capping elements or ions is different, the difference in the content of capping elements or ions between layers is 0.01 mol% or more, preferably 0.02 mol% or more, more preferably 0.03 mol% or more, even more preferably 0.04 mol% or more, particularly preferably 0.05 mol% or more.

The difference in the content of capping elements or ions between layers is preferably 3.0 mol% or less, more preferably 2.0 mol% or less, even more preferably 1.0 mol% or less, particularly preferably 0.5 mol% or less.

A certain preferred embodiment is, for example, a zirconia composite sintered body comprising zirconia, a stabilizer, at least one of Nb₂O₅ or Ta₂O₅, and a capping element or ion, and having a plurality of layers differing in the content of Nb₂O₅ or Ta₂O₅ relative to the total moles of zirconia, the stabilizer, Nb₂O₅, and Ta₂O₅,
wherein the content of Nb₂O₅ or Ta₂O₅ relative to the total moles of zirconia, the stabilizer, Nb₂O₅, and Ta₂O₅ shows an unchanging pattern of increase or decrease from one end to the other end of the zirconia composite sintered body on a straight line extending along a first direction from one end to the other end of the zirconia composite sintered body, and
the content of the capping element or ion is different between layers, and shows an unchanging pattern of increase or decrease from one end to the other end of the zirconia composite sintered body.

In view of even superior machinability in the sintered state, another example is a zirconia composite sintered body in which the content of capping elements or ions shows a pattern of increase or decrease opposite the pattern of increase or decrease in the content of Nb₂O₅ or Ta₂O₅. A specific example is a zirconia composite sintered body in which the content of capping elements or ions is in a pattern of increase when the content of Nb₂O₅ or Ta₂O₅ is in a pattern of decrease, or a zirconia composite sintered body in which the content of capping elements or ions is in a pattern of decrease when the content of Nb₂O₅ or Ta₂O₅ is in a pattern of increase. In view of providing particularly superior machinability in the sintered state, preferred is a zirconia composite sintered body in which the content of capping elements or ions is in a pattern of increase when the content of Nb₂O₅ or Ta₂O₅ is in a pattern of decrease.

In such preferred embodiments, the dental prosthesis produced can exhibit gradational translucency and an appearance more closely resembling natural teeth while possessing even superior machinability in the sintered state.

Another certain preferred embodiment is, for example, a zirconia composite sintered body comprising zirconia, a stabilizer, at least one of Nb₂O₅ or Ta₂O₅, and a capping element or ion, and having a plurality of layers that differ in the content of the stabilizer relative to the total moles of zirconia, the stabilizer, Nb₂O₅, and Ta₂O₅,
wherein the content of the stabilizer relative to the total moles of zirconia, the stabilizer, Nb₂O₅, and Ta₂O₅ shows an unchanging pattern of increase or decrease from one end to the other end of the zirconia composite sintered body on a straight line extending along a first direction from one end to the other end of the zirconia composite sintered body, and
the content of the capping element or ion is different between layers, and shows an unchanging pattern of increase or decrease from one end to the other end of the zirconia composite sintered body.

In view of even superior machinability in the sintered state, another example is a zirconia composite sintered body in which the content of capping elements or ions shows a pattern of increase or decrease unidirectionally with the pattern of increase or decrease in the content of the stabilizer. A specific example is a zirconia composite sintered body in which the content of capping elements or ions is in a pattern of decrease when the content of the stabilizer is in a pattern of decrease, or a zirconia composite sintered body in which the content of capping element or ion is in a pattern of increase when the content of the stabilizer is in a pattern of increase. In view of providing particularly superior machinability in the sintered state, preferred is a zirconia composite sintered body in which the content of capping elements or ions is in a pattern of increase when the content of the stabilizer is in a pattern of increase.

In such preferred embodiments, the dental prosthesis produced can exhibit gradational translucency and an appearance more closely resembling natural teeth while possessing even superior machinability in the sintered state.

In view of ensuring appropriate interactions between charged sites and adsorption sites at the grain boundaries, it is important that the capping elements or ions exist at the grain boundaries in the form of +1 cations or -1 anions, as noted above.

For capping elements or ions, it is preferable to use elements or ions thereof that belong to periods 2 to 7 of the periodic table, and are elements or ions thereof having a lower first ionization energy than the elements in group 18 of the same periods, or elements or ions thereof with high electron affinity. Also preferred are nitrate ions, hypochlorite ions, chlorite ions, chlorate ions, perchlorate ions, bromate ions, permanganate ions, metaborate ions, and cyanide ions.

A certain preferred embodiment is, for example, a zirconia composite sintered body in which the elements or ions thereof derived from a capping agent belong to periods 2 to 7 of the periodic table, and are elements or ions thereof having a lower first ionization energy than the elements in group 18 of the same periods, and/or elements or ions thereof with high electron affinity.

In view of facilitating the formation of +1 cations and providing even superior machinability in the sintered state, the elements belonging to periods 2 to 7 of the periodic table and having a lower first ionization energy than the elements in group 18 of the same periods are preferably Cu, Ag, Li, Na, K, Rb, Cs, and Fr, for example.

In view of facilitating the formation of -1 anions and providing even superior machinability in the sintered state, the elements with high electron affinity are preferably group 17 elements. Preferred as group 17 elements are At, I, Br, Cl, and F.

The first ionization energy refers to the energy needed to remove one electron from a neutral atom to ionize it. The first ionization energy can be referenced from Shriver & Atkins Inorganic Chemistry (Volume 1), 4th Edition, Part 1: Foundations, 1. Atomic Structure. The first ionization energy, given in eV in Appendix 2 of this book, can be converted into KJ/mol using the conversion rate of 1eV = 96.485 KJ/mol. The first ionization energy can also be determined using photoelectron yield spectroscopy (PYS).

Electron affinity (EA) is the amount of energy released when an electron is incorporated in a neutral atom. Electron affinity can be determined by the difference between the ionization potential and the energy gap. The ionization potential is defined as the energy difference between the highest occupied molecular orbital of a compound and the vacuum level, and it is measured using ultraviolet photoelectron spectroscopy.

For the first ionization energy and electron affinity, data available in the NIST Chemistry WebBook (https://webbook.nist.gov/chemistry/) may be utilized (by selecting either Ionization Energy or Electron Affinity from Ion energetics properties).

The methods described above can be appropriately employed for the measurement of first ionization energy and electron affinity because the measurement simply involves comparing the likelihood to form +1 cations or -1 anions among different elements.

Specific examples of the capping elements include Cu, Ag, Li, Na, K, Rb, Cs, Fr, At, I, Br, Cl, and F. In view of the ability to more greatly improve machinability, preferred are Cu, Ag, Li, Na, K, Rb, Cs, Fr, I, Br, Cl, and F.

A certain preferred embodiment is, for example, a zirconia composite sintered body in which the elements derived from a capping agent comprise at least one element selected from the group consisting of Cu, Ag, Li, Na, K, Rb, Cs, Fr, At, I, Br, Cl, and F, and ions of these elements are at least one +1 cation or -1 anion selected from the group consisting of Cu, Ag, Li, Na, K, Rb, Cs, Fr, I, Br, Cl, and F.

Another preferred embodiment is, for example, a zirconia composite sintered body in which the elements or ions derived from a capping agent comprise at least one element or ion thereof selected from the group consisting of Ag, Li, Na, K, Rb, Cs, Fr, At, I, Br, Cl, and F.

The capping elements or ions may be used alone, or two or more thereof may be used in combination.

As described above, the capping elements or ions act together with Nb₂O₅ and/or Ta₂O₅, and these do not compromise the effectiveness of the stabilizer. Accordingly, the present invention can exhibit its effects without particularly restricting the choice of stabilizer.

In addition to serving to coarsen the microstructure and reduce hardness, and acting together with the capping elements or ions to provide excellent free-machinability as noted above, Nb₂O₅ and Ta₂O₅ also interact with other components (for example, TiO₂, Al₂O₃) incorporated in the zirconia composite sintered body. This interaction, combined with the application of HIP, helps maximize the sintering density, providing natural tooth aesthetics.

The content of zirconia, stabilizer, Nb₂O₅, and Ta₂O₅ represents the proportion of each component in total 100 mol% of zirconia, stabilizer, Nb₂O₅, and Ta₂O₅, and the sum of zirconia, stabilizer, Nb₂O₅, and Ta₂O₅ does not exceed 100 mol%. For example, when the raw material composition contains Nb₂O₅ but does not contain Ta₂O₅, the content of zirconia, stabilizer, and Nb₂O₅ refers to the proportion of each component relative to total 100 mol% of zirconia, stabilizer, and Nb₂O₅.

In view of machinability, the ratio A/B is preferably 0.9 to 3, more preferably 0.95 to 2 in at least one of the layers in the plurality of layers, where A is the content of stabilizer in mol%, and B is the combined content of Nb₂O₅ and Ta₂O₅ in mol%. Even more preferably, the ratio A/B is 1.0 to 1.7 in view of improving free-machinability, and increasing the number of dental prostheses that can be produced in continuous processing with a single processing tool while reducing wear on processing tools.

In view of machinability, it is preferable in certain preferred embodiments that the ratio A/B is satisfied in at least a layer on the incisal-region side in the plurality of layers constituting a zirconia composite sintered body of the present invention.

In any of the foregoing embodiments, the presence of the capping agent-derived elements or ions additionally incorporated in layers satisfying the foregoing ranges of A/B leads to even superior machinability in the sintered state through the enhanced effectiveness of the combined action between the capping elements or ions and Nb₂O₅ and/or Ta₂O₅.

A certain preferred embodiment of the present invention is, for example, a zirconia composite sintered body comprising a layered structure with zirconia, a stabilizer, and at least one of Nb₂O₅ or Ta₂O₅ contained in all layers,
wherein the layered structure includes a plurality of layers that differ in the content of at least one component from among the stabilizer, Nb₂O₅, and Ta₂O₅ relative to the total moles of zirconia, the stabilizer, Nb₂O₅, and Ta₂O₅,
the layers all comprise 78 to 97.5 mol% of zirconia, 1 to 12 mol% of the stabilizer, and 1 to 9 mol% of Nb₂O₅ and Ta₂O₅ combined in total 100 mol% of zirconia, the stabilizer, Nb₂O₅, and Ta₂O₅, with a capping element or ion additionally incorporated in all layers,
the stabilizer comprises Y₂O₃ and/or CeO₂,
the content of the capping element or ion is more than 0 mol% and 5 mol% or less relative to total 100 mol% of zirconia, the stabilizer, Nb₂O₅, and Ta₂O₅, and
the zirconia composite sintered body has a ratio A/B of 0.9 to 3, where A is the content of the stabilizer in mol%, and B is the combined content of Nb₂O₅ and Ta₂O₅ in mol%.

In view of even superior strength, a certain embodiment of the present invention is, for example, a zirconia composite sintered body comprising zirconia, the stabilizer, and at least one of Nb₂O₅ or Ta₂O₅,
wherein the zirconia composite sintered body has a plurality of layers that differ in the content of at least one component from among the stabilizer, Nb₂O₅, and Ta₂O₅ relative to the total moles of zirconia, the stabilizer, Nb₂O₅, and Ta₂O₅, and comprises a zirconia enhancer in at least one of the layers of the layered structure, in addition to the zirconia, stabilizer, Nb₂O₅ and/or Ta₂O₅, and capping elements or ions.

In view of providing particularly superior strength, another example is a zirconia composite sintered body in which the zirconia enhancer is present in all of the layers in the layered structure.

In the zirconia composite sintered body comprising zirconia, the stabilizer, and Nb₂O₅ and/or Ta₂O₅, the zirconia enhancer can improve the strength of the sintered body through combined action with the capping elements or ions.

In the zirconia composite sintered body comprising a zirconia enhancer, it is also possible to appropriately modify the zirconia content, the type and content of stabilizer, the combined content of Nb₂O₅ and Ta₂O₅, the type and content of capping elements or ions, and the ratio A/B, as noted above.

When at least one of the layers in the plurality of layers constituting the zirconia composite sintered body is a layer containing a zirconia enhancer, the content of the zirconia enhancer contained therein is preferably more than 0 mass% and 6.0 mass% or less relative to total 100 mass% of zirconia, the stabilizer, and Nb₂O₅ and Ta₂O₅. In view of achieving even greater strength together with capping elements or ions when combined with capping elements or ions, the content of the zirconia enhancer is more preferably 0.01 mass% or more and 5.5 mass% or less, even more preferably 0.5 mass% or more and 5.0 mass% or less.

Examples of the zirconia enhancer include TiO₂ and Al₂O₃. The zirconia enhancer may be used alone, or two or more thereof may be used in combination.

The content of the zirconia enhancer may be the same across all layers or may be different between layers.

As used herein, "differing in the content of zirconia enhancer" means that the content of zirconia enhancer is different between a given layer and another layer when the proportion of zirconia enhancer, external to total 100 mass% of zirconia, the stabilizer, Nb₂O₅, and Ta₂O₅, is calculated separately for these layers.

In this specification, when the content of zirconia enhancer is different, the difference in the content of zirconia enhancer between layers is 0.05 mass% or more, preferably 0.08 mass% or more, more preferably 0.09 mass% or more, even more preferably 0.10 mass% or more, particularly preferably 0.11 mass% or more.

The difference in the content of zirconia enhancer between layers is preferably 3.0 mass% or less, more preferably 2.5 mass% or less, even more preferably 2.0 mass% or less, particularly preferably 1.5 mass% or less.

A certain preferred embodiment is, for example, a zirconia composite sintered body in which the zirconia enhancer comprises TiO₂, and the content of TiO₂ is 0.6 to 4.5 mass% relative to total 100 mass% of zirconia, the stabilizer, and Nb₂O₅ and Ta₂O₅.

A certain preferred embodiment is, for example, a zirconia composite sintered body comprising zirconia, a stabilizer, at least one of Nb₂O₅ or Ta₂O₅, and a capping element or ion, and having a plurality of layers that differ in the content of at least one component from among the stabilizer, Nb₂O₅, and Ta₂O₅ relative to the total moles of zirconia, the stabilizer, Nb₂O₅, and Ta₂O₅,
wherein the zirconia content is 78 to 97.5 mol%, the content of the stabilizer is 1 to 12 mol%, and the combined content of Nb₂O₅ and Ta₂O₅ is 1 to 9 mol% in total 100 mol% of zirconia, the stabilizer, Nb₂O₅, and Ta₂O₅,
the stabilizer comprises Y₂O₃ and/or CeO₂,
the zirconia enhancer comprises TiO₂, and the TiO₂ content is 0.6 to 4.5 mass% relative to total 100 mass% of zirconia, the stabilizer, and Nb₂O₅ and Ta₂O₅,
the content of the capping element or ion is more than 0 mol% and 5 mol% or less relative to total 100 mol% of zirconia, the stabilizer, Nb₂O₅, and Ta₂O₅, and
the zirconia composite sintered body has a ratio A/B of 0.9 to 3, where A is the content of the stabilizer in mol%, and B is the combined content of Nb₂O₅ and Ta₂O₅ in mol%.

A zirconia composite sintered body of the present invention has an average crystal grain size of preferably 0.5 to 5.0 µm. In view of even superior machinability, strength and translucency, the average crystal grain size is more preferably 0.5 to 4.5 µm, even more preferably 1.0 to 4.0 µm. The method of measurement of average crystal grain size is as described in the EXAMPLES below.

The average crystal grain size can be measured by adjusting the particle count in one SEM image field to about 50, 100, 200, 500, or 1,000 particles, according to the method described in the EXAMPLES below.

In the zirconia composite sintered body, higher densities result in fewer internal voids, improved translucency due to less light scattering, and enhanced strength. In view of this, the density of the zirconia composite sintered body is preferably 5.5 g/cm³ or more, more preferably 5.7 g/cm³ or more, even more preferably 5.9 g/cm³ or more.

Particularly preferably, the zirconia composite sintered body is essentially void free.

The density of the composite sintered body can be calculated by dividing its mass by its volume ((mass of composite sintered body) / (volume of composite sintered body)).

An example of a production method of a zirconia composite sintered body of the present invention is a method for producing a zirconia composite sintered body comprising the steps of fabricating a molded body using a raw material composition, and sintering the molded body,
wherein the molded body has a layered structure in which zirconia, a stabilizer, and at least one of Nb₂O₅ or Ta₂O₅ are present in all layers, and
the layered structure has a plurality of layers that differ in the content of at least one component from among the stabilizer, Nb₂O₅, and Ta₂O₅ relative to the total moles of zirconia, the stabilizer, Nb₂O₅, and Ta₂O₅.

In producing a molded body, a molded body with the intended layered structure can be obtained using a raw material composition that comprises zirconia, a stabilizer, and at least one of Nb₂O₅ or Ta₂O₅, and in which the content of at least one component from among the stabilizer, Nb₂O₅, and Ta₂O₅ is adjusted to be different between layers.

The raw material composition of the zirconia composite sintered body comprises zirconia, a stabilizer capable of preventing a phase transformation of zirconia, Nb₂O₅ and/or Ta₂O₅, and, optionally, a capping agent.

By the presence of a capping agent in the raw material composition, it is possible to even more greatly enhance the machinability improving effect produced by incorporating at least one of Nb₂O₅ or Ta₂O₅.

The raw material composition of the zirconia composite sintered body may be in a dry state, or in a liquid state containing liquid or contained in liquid. For example, the raw material composition may have a form of a powder, a granule or granulated material, a paste, or a slurry.

In an embodiment where the zirconia composite sintered body produced comprises a capping element or ion, the raw material composition comprises a capping agent. The capping agent is not particularly limited, as long as it is a compound with the potential to become a monovalent ion (+1 cation or -1 anion) in a solvent containing water. Examples include hydroxides, salts, halides (fluorides, chlorides, bromides, iodides), and cyanides containing elements or ions derived from a capping agent. The capping agent may be used alone, or two or more thereof may be used in combination.

Examples of hydroxides containing capping elements or ions include lithium hydroxide, sodium hydroxide, potassium hydroxide, rubidium hydroxide, cesium hydroxide, and francium hydroxide.

Examples of salts containing capping elements or ions include carbonates, bicarbonates, nitrates, hypochlorites, chlorites, chlorates, perchlorates, bromates, permanganates, metaborates, sulfides, and cyanides.

Examples of carbonates containing capping elements or ions include lithium carbonate, sodium carbonate, potassium carbonate, rubidium carbonate, francium carbonate, and cesium carbonate.

Examples of bicarbonates containing capping elements or ions include lithium bicarbonate, sodium bicarbonate, potassium bicarbonate, rubidium bicarbonate, francium bicarbonate, and cesium bicarbonate.

Examples of nitrates containing capping elements or ions include calcium nitrate, strontium nitrate, iron(II) nitrate, iron(III) nitrate, cobalt(II) nitrate, magnesium nitrate, gallium nitrate, yttrium(III) nitrate, lanthanum(III) nitrate, praseodymium nitrate, neodymium(III) nitrate, manganese(II) nitrate, europium nitrate, copper(II) nitrate, thorium nitrate, aluminum nitrate, nickel(II) nitrate, chromium(III) nitrate, titanium(IV) nitrate, zirconium nitrate, zirconium(IV) oxynitrate hydrate (ZrO(NO₃)₂·xH₂O), cerium(III) nitrate, tin nitrate, bismuth(III) nitrate, scandium(III) nitrate, indium(III) nitrate, and hafnium(IV) nitrate.

Examples of hypochlorites containing capping elements or ions include sodium hypochlorite, and calcium hypochlorite.

Examples of chlorites containing capping elements or ions include sodium chlorite, potassium chlorite, lithium chlorite, calcium chlorite, magnesium chlorite, barium chlorite, copper(II) chlorite, copper(III) chlorite, silver chlorite, and nickel chlorite.

Examples of chlorates containing capping elements or ions include calcium chlorate, barium chlorate, cobalt chlorate, nickel chlorate, magnesium chlorate, zinc chlorate, and copper chlorate.

Examples of perchlorates containing capping elements or ions include iron(III) perchlorate, barium perchlorate, calcium perchlorate, cobalt perchlorate, nickel perchlorate, magnesium perchlorate, beryllium perchlorate, aluminum perchlorate, and cerium perchlorate.

Examples of bromates containing capping elements or ions include neodymium bromate, lanthanum bromate, and praseodymium bromate.

Examples of permanganates containing capping elements or ions include calcium(VII) permanganate, potassium(VII) permanganate, and sodium(VII) permanganate.

Examples of metaborates containing capping elements or ions include sodium metaborate, and barium metaborate.

Examples of sulfides containing capping elements or ions include copper(I) sulfide.

Examples of cyanides containing capping elements or ions include barium cyanide, sodium cyanide, potassium cyanide, and calcium cyanide.

Examples of fluorides containing capping elements or ions include lithium fluoride, sodium fluoride, potassium fluoride, rubidium fluoride, cesium fluoride, francium fluoride, beryllium fluoride, magnesium fluoride, calcium fluoride, strontium fluoride, barium fluoride, scandium(III) fluoride, yttrium(III) fluoride, lanthanum(III) fluoride, cerium(III) fluoride, neodymium(III) fluoride, titanium(III) fluoride, titanium(IV) fluoride, zirconium(IV) fluoride, hafnium(IV) fluoride, tantalum(V) fluoride, manganese(II) fluoride, manganese(III) fluoride, iron(II) fluoride, iron(III) fluoride, copper(II) fluoride, zinc(II) fluoride, aluminum fluoride, chromium(III) fluoride, bismuth(III) fluoride, indium(III) fluoride, and tin(II) fluoride.

Examples of chlorides containing capping elements or ions include zirconium oxychloride, lithium chloride, sodium chloride, potassium chloride, rubidium chloride, cesium chloride, francium chloride, beryllium chloride, magnesium chloride, calcium chloride, strontium chloride, barium chloride, scandium(III) chloride, yttrium(III) chloride, lanthanum(III) chloride, cerium(III) chloride, praseodymium chloride, neodymium(III) chloride, samarium chloride, europium chloride, titanium(III) chloride, titanium(IV) chloride, zirconium(IV) chloride, hafnium(IV) chloride, tantalum(V) chloride, manganese chloride, iron(II) chloride, iron(III) chloride, cobalt(II) chloride, nickel(II) chloride, copper(I) chloride, copper(II) chloride, zinc(II) chloride, aluminum chloride, gallium chloride, bismuth(III) chloride, indium(I) chloride, indium(III) chloride, and tin(II) chloride.

Examples of bromides containing capping elements or ions include lithium bromide, sodium bromide, potassium bromide, rubidium bromide, cesium bromide, francium bromide, beryllium bromide, magnesium bromide, calcium bromide, strontium bromide, barium bromide, scandium(III) bromide, yttrium(III) bromide, cerium(III) bromide, neodymium(III) bromide, titanium(IV) bromide, zirconium(IV) bromide, tantalum(V) bromide, manganese(II) bromide, iron(II) bromide, iron(III) bromide, cobalt(II) bromide, nickel(II) bromide, copper(I) bromide, copper(II) bromide, zinc(II) bromide, chromium(III) bromide, bismuth(III) bromide, vanadium(III) bromide, indium(III) bromide, and tin bromide.

Examples of iodides containing capping elements or ions include iodides such as lithium iodide, sodium iodide, potassium iodide, rubidium iodide, cesium iodide, francium iodide, beryllium iodide, calcium iodide, magnesium iodide, strontium iodide, barium iodide, scandium(III) iodide, yttrium(III) iodide, lanthanum(III) iodide, cerium(III) iodide, neodymium(III) iodide, titanium(IV) iodide, zirconium(IV) iodide, hafnium(IV) iodide, tantalum(V) iodide, manganese(II) iodide, iron(II) iodide, iron(III) iodide, cobalt(II) iodide, nickel(II) iodide, copper(I) iodide, zinc(II) iodide, aluminum iodide, chromium(III) iodide, vanadium(II) iodide, bismuth(III) iodide, indium(III) iodide, tin(II) iodide, and tin(IV) iodide.

Commercially available zirconia powders can be used for zirconia.

Examples of such commercially available products include zirconia powders manufactured by Tosoh Corporation under the trade names Zpex^{®} (Y₂O₃ content: 3 mol%), Zpex^{®} 4 (Y₂O₃ content: 4 mol%), Zpex^{®} Smile^{®} (Y₂O₃ content: 5.5 mol%), TZ-3Y (Y₂O₃ content: 3 mol%), TZ-3YS (Y₂O₃ content: 3 mol%), TZ-4YS (Y₂O₃ content: 4 mol%), TZ-6Y (Y₂O₃ content: 6 mol%), TZ-6YS (Y₂O₃ content: 6 mol%), TZ-8YS (Y₂O₃ content: 8 mol%), TZ-10YS (Y₂O₃ content: 10 mol%), TZ-3Y-E (Y₂O₃ content: 3 mol%), TZ-3YS-E (Y₂O₃ content: 3 mol%), TZ-3YB-E (Y₂O₃ content: 3 mol%), TZ-3YSB-E (Y₂O₃ content: 3 mol%), TZ-3YB (Y₂O₃ content: 3 mol%), TZ-3YSB (Y₂O₃ content: 3 mol%), TZ-3Y20AB (Y₂O₃ content: 3 mol%), TZ-8YSB (Y₂O₃ content: 8 mol%), and TZ-0 (Y₂O₃ content: 0 mol%). These commercially available zirconia powders also contain HfO₂. It is possible to use commercially available products additionally containing Y₂O₃.

The raw material composition of the present invention may use zirconia powders in which Y₂O₃ is uniformly dispersed as a solid solution, as in the TZ series of the commercially available products listed above (those with "TZ" in the product names).

The method of production of zirconia powder is not particularly limited, and known methods may be employed, for example, such as the breakdown process, where coarse particles are pulverized into fine powder, or the building-up process, where synthesis occurs through nucleation and growth from atoms or ions.

The type of zirconia powder in the raw material composition is not particularly limited. It is possible to additionally incorporate stabilizer particles when the zirconia powder comprises zirconia but does not contain a stabilizer, or when increasing the content of stabilizer as needed. The stabilizer particles are not particularly limited, as long as the content of the stabilizer within the zirconia composite sintered body can be adjusted to the predetermined ranges mentioned above.

For example, the stabilizer particles may be commercially available products, and may be prepared by pulverizing a commercially available powder using a known pulverizing mixer (such as a ball mill).

The stabilizer may be either a stabilizer not dissolved in zirconia as a solid solution, or a stabilizer dissolved in zirconia as a solid solution.

In view of ease of obtaining the desired zirconia composite sintered body, a certain preferred embodiment is, for example, a method for producing a zirconia composite sintered body in which the stabilizer (preferably, Y₂O₃) in the raw material composition comprises a stabilizer not dissolved in zirconia as a solid solution. The presence of stabilizers not dissolved in zirconia as a solid solution can be verified, for example, through X-ray diffraction (XRD) patterns.

The presence of peaks derived from the stabilizer in an XRD pattern of the raw material composition or molded body indicates the presence of a stabilizer that is not dissolved in zirconia in the raw material composition or molded body.

A peak derived from the stabilizer is basically not observable in an XRD pattern when the stabilizer is fully dissolved in zirconia as a solid solution. It is, however, possible, depending on the crystal state or other conditions of the stabilizer, that the stabilizer is not dissolved in zirconia as a solid solution even when the XRD pattern does not show peaks for stabilizers.

The following describes situations where the stabilizer comprises a stabilizer not dissolved in zirconia as a solid solution, with yttria serving as an example of the stabilizer.

In the raw material composition or molded body of the present invention, the abundance f_{y} of yttria not dissolved in zirconia as a solid solution (hereinafter, also referred to as "undissolved yttria") can be calculated using the following mathematical formula (1).${f}_{y} = {I}_{29} / \left({I}_{28} + {I}_{29} + {I}_{30}\right) \times 100$ (1) where f_{y} represents the fraction (%) of undissolved yttria, I₂₈ represents the integrated intensity of a peak near 2θ = 28°, where the main peak of monoclinic zirconia appears in XRD measurement, I₂₉ represents the integrated intensity of a peak near 2θ = 29°, where the main peak of yttria appears in XRD measurement, and I₃₀ represents the integrated intensity of a peak near 2θ = 30°, where the main peak of tetragonal or cubic zirconia appears in XRD measurement.

When additionally using stabilizers other than yttria, the formula can be applied to calculate the abundance of undissolved stabilizers other than yttria by substituting I₂₉ with the peaks of other stabilizers.

In view of considerations such as ease of obtaining the desired zirconia composite sintered body, the abundance f_{y} of undissolved yttria is preferably higher than 0%, more preferably 1% or more, even more preferably 2% or more, particularly preferably 3% or more. The upper limit for the abundance f_{y} of undissolved yttria may be, for example, 25% or less. Preferably, the upper limit of abundance f_{y} of undissolved yttria depends on the yttria content in the raw material composition or molded body.

For example, the abundance f_{y} of undissolved yttria falls within the following ranges when the yttria content in the raw material composition or molded body of the present invention is 3 mol% or more and 8 mol% or less.

When the yttria content is 3 mol% or more and less than 4.5 mol%, f_{y} may be 15% or less. When the yttria content is 4.5 mol% or more and less than 5.8 mol%, f_{y} may be 20% or less. When the yttria content is 5.8 mol% or more and 8 mol% or less, f_{y} may be 25% or less.

For example, when the yttria content is 3 mol% or more and less than 4.5 mol%, f_{y} is preferably 2% or more, more preferably 3% or more, even more preferably 4% or more, particularly preferably 5% or more.

When the yttria content is 4.5 mol% or more and less than 5.8 mol%, f_{y} is preferably 3% or more, more preferably 4% or more, even more preferably 5% or more, yet more preferably 6% or more, particularly preferably 7% or more.

When the yttria content is 5.8 mol% or more and 8 mol% or less, f_{y} is preferably 4% or more, more preferably 5% or more, even more preferably 6% or more, yet more preferably 7% or more, particularly preferably 8% or more.

In the raw material composition or molded body of the present invention, it is not necessarily required for the stabilizer to be fully dissolved in zirconia as a solid solution. In the present invention, "stabilizer being dissolved as a solid solution" means that, for example, the elements (atoms) contained in the stabilizer are dissolved in zirconia as a solid solution.

There is no particular restriction on Nb₂O₅ and/or Ta₂O₅ incorporated in the raw material composition of the present invention, as long as the content of Nb₂O₅ and/or Ta₂O₅ within the zirconia composite sintered body can be adjusted in the predetermined ranges mentioned above. For example, commercially available products may be used for Nb₂O₅ and/or Ta₂O₅ without any particular restrictions. These may be used after pulverizing its powder using a known pulverizing mixer (such as a ball mill).

An example method of obtaining the raw material composition involves a process whereby the raw material composition is prepared by, for example, wet mixing each raw material of the raw material composition (zirconia, a stabilizer, Nb₂O₅ and/or Ta₂O₅, and, optionally, a capping agent (for example, a compound with the potential to become a monovalent ion in a solvent containing water) and, additionally, a zirconia enhancer) in a solvent containing water.

The method for wet mixing the raw materials in a solvent containing water is not particularly limited. For example, the raw materials may be formed into a slurry through wet pulverization and mixing using a known pulverizing mixer (such as a ball mill). The slurry can then be dried and granulated to produce a granular raw material composition.

In the wet mixing process, it is possible to additionally include additives such as binders, plasticizers, dispersants, emulsifiers, antifoaming agents, pH adjusters, and lubricants. The additives may be used alone, or two or more thereof may be used in combination.

The binder may be added to the pulverized slurry after the slurry is formed by adding a primary powder mixture of zirconia, Y₂O₃, Nb₂O₅ and/or Ta₂O₅, and, optionally, a capping agent into water.

The binder is not particularly limited, and known binders may be used. Examples of the binders include polyvinyl alcohol binders, acrylic binders, wax binders (such as paraffin wax), methyl cellulose, carboxymethyl cellulose, polyvinyl butyral, polymethylmethacrylate, ethyl cellulose, polyethylene, polypropylene, ethylene-vinyl acetate copolymer, polystyrene, atactic polypropylene, and methacrylic resin.

Examples of the plasticizers include polyethylene glycol, glycerin, propylene glycol, and dibutyl phthalic acid.

Examples of the dispersants include ammonium polycarboxylates (such as triammonium citrate), ammonium polyacrylate, acrylic copolymer resin, acrylic acid ester copolymer, polyacrylic acid, bentonite, carboxymethyl cellulose, anionic surfactants (for example, polyoxyethylene alkyl ether phosphoric acid esters such as polyoxyethylene lauryl ether phosphoric acid ester), non-ionic surfactants, oleic glyceride, amine salt surfactants, oligosaccharide alcohols, and stearic acid.

Examples of the emulsifiers include alkyl ethers, phenyl ethers, and sorbitan derivatives.

Examples of the antifoaming agents include alcohols, polyethers, silicone, and waxes.

Examples of the pH adjusters include ammonia, and ammonium salts (including ammonium hydroxides such as tetramethylammonium hydroxide).

Examples of the lubricants include polyoxyethylene alkyl ethers, and waxes.

The solvent used for wet mixing is not particularly limited, as long as it contains water. By using organic solvents, the solvent may be a mixed solvent of water and organic solvent. Alternatively, the solvent may be solely water. Examples of the organic solvents include ketone solvents such as acetone, and ethyl methyl ketone; and alcohol solvents such as ethanol, 1-propanol, 2-propanol, 2-methyl-2-propanol, glycerin, diglycerin, polyglycerin, propylene glycol, dipropylene glycol, polypropylene glycol, ethylene glycol, diethylene glycol, polyethylene glycol, polyethylene glycol monomethyl ether, 1,2-pentadiol, 1,2-hexanediol, and 1,2-octanediol.

The raw material composition used for the zirconia composite sintered body in the present invention may optionally comprise other additional components aside from a capping agent and a zirconia enhancer, in addition to zirconia, Y₂O₃, Nb₂O₅, and Ta₂O₅, provided that the present invention can exhibit its effects. Examples of such additional components include colorants (pigments and composite pigments), fluorescent agents, and SiO₂. The additional components may be used alone, or two or more thereof may be used in combination.

Examples of the pigments include oxides of at least one element selected from the group consisting of V, Cr, Mn, Fe, Co, Ni, Zn, Y, Zr, Sn, Sb, Bi, Ce, Pr, Sm, Eu, Gd, Tb, and Er (specifically, such as NiO, and Cr₂O₃), preferably oxides of at least one element selected from the group consisting of V, Cr, Mn, Fe, Co, Ni, Zn, Y, Zr, Sn, Sb, Bi, Ce, Pr, Sm, Eu, Gd, and Tb, more preferably oxides of at least one element selected from the group consisting of V, Cr, Mn, Fe, Co, Ni, Zn, Y, Zr, Sn, Sb, Bi, Ce, Sm, Eu, Gd, and Tb. The pigments may exclude Y₂O₃ and CeO₂.

Examples of the composite pigments include (Zr,V)O₂, Fe(Fe,Cr)₂O₄, (Ni,Co,Fe)(Fe,Cr)₂O₄·ZrSiO₄, and (Co,Zn)Al₂O₄.

Examples of the fluorescent agents include Y₂SiO₅:Ce, Y₂SiO₅:Tb, (Y,Gd,Eu)BO₃, Y₂O₃:Eu, YAG:Ce, ZnGa₂O₄:Zn, and BaMgAl₁₀O₁₇:Eu.

Following its production, the raw material composition is molded to fabricate a molded body. The molding method is not particularly limited, and known molding methods can be used (for example, such as press molding).

When producing a zirconia molded body using a method that includes press molding the raw material composition, there are no specific limitations on the press molding method, and press molding may be achieved using known press molding machines. Specific examples of press molding methods include uniaxial pressing.

The pressure in press molding is appropriately optimized according to the desired size of molded body, open porosity, biaxial flexural strength, and the particle size of raw material powder. Typically, the pressure in press molding ranges from 5 MPa to 1,000 MPa. By increasing the molding pressure in the method of production above, it is possible to achieve more effective closure of pores in the resulting molded body, leading to a lower open porosity and increasing the density of the molded body. To increase the density of the zirconia molded body obtained, cold isostatic pressing (CIP) may be applied after uniaxial pressing.

Following its production, the molded body is sintered to obtain a zirconia composite sintered body.

The sintering temperature (highest sintering temperature) for sintering the molded body to obtain the zirconia composite sintered body is, for example, preferably 1,300°C or more, more preferably 1,350°C or more, even more preferably 1,400°C or more, yet more preferably 1,450°C or more, particularly preferably 1,500°C or more. The sintering temperature is, for example, preferably 1,680°C or less, more preferably 1,650°C or less, even more preferably 1,600°C or less. Preferably, the method for producing a zirconia composite sintered body of the present invention comprises the step of sintering the molded body at a highest sintering temperature of 1,300 to 1,680°C. The highest sintering temperature is preferably a temperature in the atmosphere.

The hold time (retention time) at the highest sintering temperature is preferably 30 hours or less, more preferably 20 hours or less, even more preferably 10 hours or less, yet more preferably 5 hours or less, particularly preferably 3 hours or less, most preferably 2 hours or less, depending on the temperature. The hold time may be 25 minutes or less, 20 minutes or less, or 15 minutes or less. The hold time is preferably 1 minute or more, more preferably 5 minutes or more, even more preferably 10 minutes or more. A production method of the present invention enables the fabrication of a zirconia composite sintered body having superior flexural strength and translucency and excellent machinability in the sintered state in a manner that depends on the content of the stabilizer. The sintering time can be reduced, as long as the present invention can exhibit its effects. Shortening the sintering time can improve production efficiency and reduce energy costs.

In the method for producing a zirconia composite sintered body of the present invention, the rate of temperature increase in sintering the molded body is not particularly limited, and is preferably 0.1°C/min or more, more preferably 0.2°C/min or more, even more preferably 0.5°C/min or more. The rate of temperature increase is preferably 50°C/min or less, more preferably 30°C/min or less, even more preferably 20°C/min or less. Productivity improves when the rate of temperature increase is at or above these lower limits.

A common dental zirconia furnace can be used for the sintering process of the molded body. The dental zirconia furnace may be a commercially available product. Examples of commercially available products include Noritake KATANA^{®} F-1, F-1N, F-2 (SK Medical Electronics Co., Ltd.).

Preferably, the sintering process for the molded body includes a hot isostatic pressing (HIP) process, aside from sintering at the highest sintering temperature. The HIP process can further improve the translucency and strength of the zirconia composite sintered body.

In the following, the term "primary sintered body" is used to refer to sintered bodies obtained through sintering at the highest sintering temperature, whereas "HIP sintered body" is used to refer to sintered bodies after a HIP process.

Known hot isostatic pressing (HIP) devices can be used for the HIP process.

The temperature of the HIP process is not particularly limited. However, for advantages such as obtaining a high-strength and dense zirconia composite sintered body, the HIP temperature is preferably 1,200°C or more, more preferably 1,300°C or more, even more preferably 1,400°C or more. The HIP temperature is preferably 1,700°C or less, more preferably 1,650°C or less, even more preferably 1,600°C or less.

In the method for producing a zirconia composite sintered body of the present invention, the HIP pressure in the HIP process of the primary sintered body is not particularly limited. However, for advantages such as obtaining a high-strength and dense sintered body, the HIP pressure is preferably 100 MPa or more, more preferably 125 MPa or more, even more preferably 130 MPa or more. The upper limit of HIP pressure is not particularly limited, and may be, for example, 400 MPa or less, 300 MPa or less, or 200 MPa or less.

In the method for producing a zirconia composite sintered body of the present invention, the rate of temperature increase in the HIP process of the primary sintered body is not particularly limited, and is preferably 0.1°C/min or more, more preferably 0.2°C/min or more, even more preferably 0.5°C/min or more. The rate of temperature increase is preferably 50°C/min or less, more preferably 30°C/min or less, even more preferably 20°C/min or less. Productivity improves when the rate of temperature increase is at or above these lower limits.

In the method for producing a zirconia composite sintered body of the present invention, the duration of HIP in the HIP process of the primary sintered body is not particularly limited. However, for advantages such as obtaining a high-strength and dense zirconia composite sintered body, the duration of the HIP process is preferably 5 minutes or more, more preferably 10 minutes or more, even more preferably 30 minutes or more. The duration of the HIP process is preferably 10 hours or less, more preferably 6 hours or less, even more preferably 3 hours or less.

In the method for producing a zirconia composite sintered body of the present invention, the pressure medium in the HIP process of the primary sintered body is not particularly limited. However, in view of a low impact on zirconia, the pressure medium may be at least one selected from the group consisting of oxygen gas, oxygen-mixed gas, air, and inert gas (for example, such as nitrogen gas, argon gas).

When conducting HIP processing of the primary sintered body in an oxygen-mixed gas atmosphere, the oxygen concentration may be, for example, more than 0% and 20% or less, though it is not particularly limited.

When using an oxygen-mixed gas, at least one inert gas (for example, such as nitrogen gas or argon gas) may be selected as a gas other than oxygen.

In a method for producing a zirconia composite sintered body of the present invention, the HIP process may result in blackening due to oxygen deficiency when the process is carried out in a reducing atmosphere such as by using an inert gas. In order to prevent such blackening, it is preferable to include a heat treatment step (or tempering as it is also called hereinbelow), performed at 1,650°C or less in either the atmosphere or an excess oxygen atmosphere, after the HIP process. In view of the efficiency of heat treatment, the heat treatment step is carried out preferably in an excess oxygen atmosphere. Here, "excess oxygen atmosphere" means an atmosphere where the oxygen concentration exceeds that of the atmosphere. The excess oxygen atmosphere is not particularly limited, as long as the oxygen concentration is higher than 21% and 100% or less, and can be appropriately selected within this range. For example, the oxygen concentration may be 100%.

In the method for producing a zirconia composite sintered body of the present invention, the conditions employed in the HIP process may be appropriately adjusted to adjust translucency. For example, it is possible to deliberately reduce translucency by lowering the heat-treatment temperature. That is, in addition to making adjustments by means such as adjusting components, the conditions employed in the HIP process may be adjusted to achieve the desired translucency in each layer, such as by reducing translucency in a layer corresponding to the cervical region and increasing translucency in a layer corresponding to the incisal region.

A zirconia composite sintered body of the present invention may be a primary sintered body, a HIP sintered body, or a tempered sintered body with no particular restriction, provided that the present invention can exhibit its effects.

A certain preferred embodiment is, for example, a zirconia composite sintered body that has been tempered.

The heat treatment temperature in the atmosphere or an excess oxygen atmosphere may be appropriately varied according the aesthetics of the zirconia composite sintered body (for example, the shade of dental prostheses).

In a certain preferred embodiment, in view of the aesthetics of the zirconia composite sintered body, the heat treatment temperature in the atmosphere or an excess oxygen atmosphere is preferably 1,650°C or less, more preferably 1,600°C or less, even more preferably 1,550°C or less.

In another preferred embodiment, in view of the aesthetics of the zirconia composite sintered body, the heat treatment temperature in the atmosphere or an excess oxygen atmosphere is preferably 1,400°C or less, more preferably 1,300°C or less, even more preferably 1,200°C or less.

In either embodiment, the heat treatment temperature is preferably 500°C or more, more preferably 600°C or more, even more preferably 700°C or more.

A common dental zirconia furnace can be used for the tempering process. The dental zirconia furnace may be a commercially available product. Examples of commercially available products include Noritake KATANA^{®} F-1, F-1N, F-2 (SK Medical Electronics Co., Ltd.).

A zirconia composite sintered body of the present invention exhibits excellent machinability despite being a sintered body. It is accordingly not necessary to machine it as a mill blank of a composite pre-sintered body in a semi-sintered state before sintering into a sintered body.

Nonetheless, the zirconia composite sintered body can be produced using a method whereby a molded body obtained from the raw material composition is pre-sintered to form a semi-sintered-state composite pre-sintered body, and this composite pre-sintered body, unprocessed, is machined before sintering it to form a sintered body.

Another certain embodiment is, for example, a method for producing a zirconia composite sintered body that comprises the steps of fabricating a molded body using the raw material composition, pre-sintering the molded body to form a zirconia composite pre-sintered body (pre-sintering step), and sintering the zirconia composite pre-sintered body.

In order to ensure block formation, the firing temperature (pre-sintering temperature) in the pre-sintering step is, for example, preferably 800°C or more, more preferably 900°C or more, even more preferably 950°C or more.

The pre-sintering temperature is, for example, preferably 1,200°C or less, more preferably 1,150°C or less, even more preferably 1,100°C or less. The preferred range of pre-sintering temperatures is, for example, 800°C to 1,200°C. It is believed that, at these pre-sintering temperatures, no significant progression of the formation of the solid solution of the stabilizer occurs in the pre-sintering step.

The zirconia composite pre-sintered body has a density of preferably 2.7 g/cm³ or more. The zirconia composite pre-sintered body has a density of preferably 4.0 g/cm³ or less, more preferably 3.8 g/cm³ or less, even more preferably 3.6 g/cm³ or less. Processing becomes easier when the density falls within these ranges. The density of the composite pre-sintered body can be calculated, for example, as the mass-to-volume ratio of the composite pre-sintered body ((mass of composite pre-sintered body) / (volume of composite pre-sintered body)).

The zirconia composite pre-sintered body has a three-point flexural strength of preferably 15 to 70 MPa, more preferably 18 to 60 MPa, even more preferably 20 to 50 MPa.

The flexural strength can be measured in compliance with ISO 6872:2015 except for the specimen size, using a specimen measuring 5 mm in thickness, 10 mm in width, and 50 mm in length. For surface finishing, the specimen surfaces, including chamfered surfaces (45° chamfers at the corners of specimen), are finished longitudinally with #600 sandpaper. The specimen is disposed in such an orientation that its widest face is perpendicular to the vertical direction (loading direction). In the flexure test, measurements are made at a span of 30 mm with a crosshead speed of 1.0 mm/min.

The sintering step for the zirconia composite pre-sintered body can be conducted using the same method and conditions (such as temperature and pressure) used to sinter the molded body. Accordingly, in embodiments of the production method using a zirconia composite pre-sintered body, the term "molded body" can be read as referring to "composite pre-sintered body".

A zirconia composite sintered body of the present invention excels in strength. A zirconia composite sintered body of the present invention has a biaxial flexural strength of preferably 300 MPa or more, more preferably 350 MPa or more, even more preferably 400 MPa or more, yet more preferably 450 MPa or more, particularly preferably 500 MPa or more. A zirconia composite sintered body of the present invention, with its biaxial flexural strength falling in these ranges, can reduce defects, for example, such as fracture in the oral cavity, when used as a dental prosthesis. The upper limit of biaxial flexural strength is not particularly limited, and may be, for example, 1,200 MPa or less, or 1,000 MPa or less. The biaxial flexural strength of the zirconia composite sintered body can be measured in compliance with ISO 6872:2015.

It is preferable that a zirconia composite sintered body of the present invention have high translucency. The translucency can be evaluated using ΔL*(W-B).

Specifically, a zirconia composite sintered body of the present invention, in each of its layers within the layered structure, has a translucency of preferably 10 or more, more preferably 11 or more, even more preferably 12 or more, particularly preferably 13 or more in terms of a ΔL*(W-B) in a sample measuring 15 mm in diameter and 1.2 mm in thickness.

With ΔL*(W-B) falling within these ranges, and by the presence of a plurality of layers that differ in the content of at least one component from among a stabilizer, Nb₂O₅, and Ta₂O₅, it is possible to provide gradational translucency across the layers, with a gradation that is sufficient even in comparison with natural teeth, resulting in a highly aesthetic zirconia composite sintered body more closely resembling natural teeth.

Here, ΔL*(W-B) means the difference between the lightness (first L* value) against a white background and the lightness (second L* value) against a black background in the same sample. Specifically, ΔL*(W-B) means the difference between the L* value against a white background (JIS Z 8781-4:2013 Color Measurements - Part 4: CIE 1976 L*a*b* color space) and the L* value against a black background. The white background means the white part of the hiding-power test paper described in JIS K 5600-4-1:1999, Part 4, Section 1, and the black background means the black part of the hiding-power test paper.

The upper limit of ΔL*(W-B) is not particularly limited, and may be, for example, 25 or less. In view of aesthetics, ΔL*(W-B) may be 20 or less.

A spectrophotometer can be used to measure ΔL* for a zirconia composite sintered body with a diameter of 15 mm and a thickness of 1.2 mm. For example, the dental colorimeter Crystaleye CE100-CE/JP with a 7-band LED illuminant and analysis software Crystaleye (manufactured by Olympus Corporation) may be used for measurement.

Examples of dental prostheses produced using a zirconia composite sintered body of the present invention include crown restorations such as inlays, onlays, veneers, crowns, core-integrated crowns, and bridges, as well as abutment teeth, dental posts, dentures, denture bases, and implant parts (fixtures, abutments).

Preferably, for example, a commercially available dental CAD/CAM system is used for machining. Examples of such CAD/CAM systems include the CEREC system manufactured by Dentsply Sirona, and the KATANA^{®} system manufactured by Kuraray Noritake Dental Inc.

A zirconia composite sintered body of the present invention can also be used in applications other than dental use, and is particularly suited for zirconia parts that require irregular or complex shapes and strength.

Compared to sintered bodies produced solely by existing production methods (such as injection molding, CIP, casting, and 3D printing), a zirconia composite sintered body of the present invention can be directly processed as a sintered body. This can be cost-effective, for example, when a desired zirconia part is obtained in short time periods. For parts that are complex in shape and difficult to produce with traditional methods, this technique eliminates the need for mechanical fitting of multiple parts, allowing for the production of zirconia parts with maintained high strength. Furthermore, because the sintered body is directly processable, the sintering process can be eliminated when precise dimensions are needed, eliminating uneven shrinkage during firing, leading to high-precision zirconia parts. A zirconia composite sintered body of the present invention is also applicable to methods of manufacture in applications, specifically, such as in jewelry goods, engine parts and interior components of mobility vehicles such as aircraft and automobiles, display panel frames, construction members, electrical appliance components, components of household goods, and toy parts.

The zirconia parts can also be fitted with different materials to create composite components.

The present invention encompasses embodiments combining all or part of the foregoing features and embodiments in various forms, provided that the present invention can exhibit its effects with such combinations within the technical idea of the present invention.

### EXAMPLES

The present invention is described below in greater detail through Examples. It is to be noted, however, that the present invention is in no way limited by the following Examples, and various modifications may be made by a person with ordinary skill in the art within the technical idea of the present invention.

In the following Examples and Comparative Examples, the average particle diameter refers to an average primary particle diameter, and can be determined using a laser diffraction scattering method. Specifically, the average particle diameter can be measured by volume with ultrasonic waves being applied after a slurry diluted with water is subjected to 30 minutes of ultrasonication, using a laser diffraction/scattering particle size distribution analyzer (Partica LA-950 manufactured by Horiba Ltd.).

### [Examples 1 to 11 and Comparative Examples 1 and 2]

The measurement samples for Examples and Comparative Examples were prepared through processes including the fabrication a granular raw material composition, a molded body, and a zirconia composite sintered body (the fabrication of a primary sintered body, as well as HIP processing and tempering).

### [Fabrication of Granular Raw Material Composition]

To fabricate a granular raw material composition for each Example and Comparative Example, a mixture was prepared by combining commercially available powders of ZrO₂, Y₂O₃, Nb₂O₅, and Ta₂O₅, along with a capping agent and a TiO₂ powder, so as to yield, after sintering, a zirconia composite sintered body with the content of each component indicated in Tables 1 and 2. Subsequently, water was added to prepare a slurry, and the slurry was pulverized by wet ball milling until it had an average particle diameter of 0.13 µm or less. After adding a binder to the pulverized slurry, the slurry was dried with a spray dryer to obtain a granular raw material composition (hereinafter, also referred to simply as "raw material composition"). This raw material composition was used to produce a molded body, as detailed below.

The average particle diameter was measured by volume with ultrasonic waves being applied after the slurry diluted with water was subjected to 30 minutes of ultrasonication, using a laser diffraction/scattering particle size distribution analyzer (Partica LA-950 manufactured by Horiba Ltd.).

### [Fabrication of Molded Body]

For each Example and Comparative Example, pellet-shaped molded bodies and block-shaped molded bodies were produced in preparation for the fabrication of zirconia composite sintered body samples for both translucency and strength evaluation and processability evaluation, as follows.

A cylindrical mold with a diameter of 19 mm was used to form pellet-shaped molded bodies. The raw material composition was placed in the mold to ensure that sintering produces a zirconia composite sintered body with a thickness of 1.2 mm.

Subsequently, the raw material composition was press molded under a surface pressure of 200 MPa to obtain a pellet-shaped molded body, using a uniaxial press molding machine.

For block-shaped molded bodies, the raw material composition was placed in a mold with inside dimensions of 19 mm × 18 mm in the sequence indicated in Tables 1 and 2, ensuring that sintering produces a zirconia composite sintered body with a height of 14.5 mm. Here, the raw material composition was placed in equal amounts for each layer. Subsequently, the raw material composition was press molded for 90 seconds under a surface pressure of 200 MPa to obtain a molded body having a layered structure, using a uniaxial press molding machine.

### [Fabrication of Primary Sintered Body]

The pellet-shaped and block-shaped molded bodies were held for 2 hours in the atmosphere at the highest sintering temperatures specified in Tables 1 and 2, using the Noritake KATANA^{®} F-1 furnace manufactured by SK Medical Electronics Co., Ltd. This resulted in zirconia composite sintered body (primary sintered body) samples in both pellet and block shapes.

### [Fabrication of HIP Sintered Body]

The pellet-shaped and block-shaped zirconia composite sintered bodies (primary sintered bodies) were held for 2 hours in an argon atmosphere at 150 MPa and the HIP temperatures specified in Tables 1 and 2, using the HIP device O₂-Dr.HIP manufactured by Kobe Steel, Ltd. This resulted in zirconia composite sintered body (HIP sintered body) samples in both pellet and block shapes.

### [Fabrication of Zirconia Composite Sintered Body (Tempered Sintered Body)]

The pellet-shaped and block-shaped zirconia composite sintered bodies (HIP sintered bodies) were held at 700°C for 60 hours using the Noritake KATANA^{®} F-1 furnace (manufactured by SK Medical Electronics Co., Ltd.) to prepare zirconia composite sintered body (tempered sintered body) samples in both pellet and block shapes. The pellet-shaped samples had a diameter of 15 mm and a thickness of 1.2 mm. The block-shaped samples measured 15.7 mm in width, 16.5 mm in length, and 14.5 mm in height.

The content of each component in the zirconia composite sintered bodies presented in Tables 1 and 2 was calculated from the quantities of the raw materials charged.

The content (mol%) of capping elements or ions (e.g., Na) in Tables 1 and 2 refers to the proportion external to total 100 mol% of zirconia, the stabilizer (yttria), Nb₂O₅, and Ta₂O₅.

The content (mol%) of zirconia, the stabilizer, Nb₂O₅, and Ta₂O₅ in Tables 1 and 2 refers to the content of each component in total 100 mol% of zirconia, the stabilizer, Nb₂O₅, and Ta₂O₅.

The content (mass%) of TiO₂ in Tables 1 and 2 refers to the proportion external to total 100 mass% of zirconia, the stabilizer, Nb₂O₅, and Ta₂O₅.

A/B in Tables 1 and 2 represents the ratio of A to B, where A is the Y₂O₃ content in mol%, and B is the combined content of Nb₂O₅ and Ta₂O₅ in mol%.

### [Translucency Evaluation of Zirconia Composite Sintered Body]

The pellet-shaped zirconia composite sintered body (tempered sintered body) samples (approximately 15 mm in diameter × 1.2 mm thickness) of each Example and Comparative Example were directly used to evaluate translucency, as follows (n = 3).

The dental colorimeter Crystaleye (7-band LED illuminant) manufactured by Olympus Corporation was used as the measurement device. First, a white background (underlay) was arranged for the sample (the opposite side of the sample from the measurement device is white), and the sample was measured for L* value according to L*a*b*color system (JIS Z 8781-4:2013 Color Measurements - Part 4: CIE 1976 L*a*b* color space). This was recorded as a first L* value.

Thereafter, the same sample used for the measurement of first L* value was measured for L* value against a black background (underlay) according to L*a*b*color system (the opposite side of the sample from the measurement device is black). This was recorded as a second L* value.

In the present invention, the difference between the first and second L* values (the second L* value is subtracted from the first L* value) represents translucency, denoted as ΔL*(W-B).

Higher values of ΔL*(W-B) indicate higher translucency, whereas lower ΔL* values indicate lower translucency.

A hiding-power test paper used for measurement involving paints, as specified in JIS K 5600-4-1:1999, was used for the black and white backgrounds (underlays) in the chromaticity measurement. The arithmetic mean value of ΔL*(W-B) for each sample is presented in Tables 1 and 2.

### [Aesthetic Evaluation of Zirconia Composite Sintered Body]

The samples with the shape of an anterior tooth crown used in the [Processability Evaluation of Zirconia Composite Sintered Body] below were visually observed from a close distance (approximately 10 cm from the sample) (n = 3).

The zirconia composite sintered body was considered acceptable (O) when the tone (a sense of transparency) appeared comparable to that of natural teeth in all three samples.

The zirconia composite sintered body was considered unacceptable (×) when the tone (a sense of transparency) did not appear comparable to that of natural teeth in all three samples.

In Comparative Example 1, the sintered body was unmachinable, as indicated in the result of processability evaluation in Table 2. Accordingly, in Comparative Example 1, a pre-sintered body obtained after retaining the block-shaped molded body at 1,000°C for 2 hours was processed into the shape of an anterior tooth crown, and this pre-sintered body was retained at 1,550°C for 2 hours to obtain a crown-shaped sample for the evaluation of aesthetics.

### [Strength Evaluation of Zirconia Composite Sintered Body]

The pellet-shaped zirconia composite sintered body (tempered sintered body) samples of each Example and Comparative Example were directly used to measure biaxial flexural strength. The measurement was conducted using a universal testing machine AGS-X (manufactured by Shimadzu Corporation) with the crosshead speed set at 1.0 mm/min, following ISO6872:2015 (n = 5). The measurement results are presented as arithmetic mean values in Tables 1 and 2. A strength of 300 MPa or more was considered acceptable.

### [Measurement Method for Average Crystal Grain Size of Sintered Body]

Surface images were captured for the pellet-shaped zirconia composite sintered body (tempered sintered body) of each Example and Comparative Example, using a scanning electron microscope (VE-9800 manufactured by Keyence under this trade name). The average crystal grain size was calculated by image analysis after indicating grain boundaries on individual crystal grains in the image. The results are presented in Tables 1 and 2.

For the measurement of average crystal grain size, the captured SEM image was binarized with image analysis software (Image-Pro Plus manufactured by Hakuto Co., Ltd. under this trade name), and particles were recognized from the field (region) by adjusting the brightness range to provide clear grain boundaries. The crystal grain size from Image-Pro Plus is the average of the measurements of the length of a line segment connecting the contour line and passing through the centroid determined from the contour line of the crystal grain, conducted at 2-degree intervals with the centroid as the central point. The measurement of crystal grain size was conducted for all particles not lying on the edges of the SEM photographic image (3 fields) of each Example and Comparative Example. The arithmetic average of the measured crystal grain sizes was then determined as the average crystal grain size (number-based) of the sintered body.

Note that "particles not lying on the edges of the image" refers to particles excluding those with contour lines extending beyond the frame of the SEM photographic image (particles with their contour lines interrupted by the boundary lines at the top, bottom, left, and right). To select the crystal grain size of all particles not lying on the edges of the image, the option in Image-Pro Plus was used that excludes all particles lying on the boundary lines.

### [Processability Evaluation of Zirconia Composite Sintered Body]

For each Example and Comparative Example, 30 samples of block-shaped zirconia composite sintered bodies (tempered sintered bodies) were prepared, each with a metal fixture attached to a surface 15.7 mm in width and 14.5 mm in height. These samples were then processed into the shape of a typical anterior tooth crown using the CEREC system MC-XL manufactured by Dentsply Sirona. For the machining program, inLab^{®} CAM version 20.0.1.203841 software was used with the following settings: Manufacture: IVOCLAR VIVADENT, Material name: IPS e.max CAD, Production Method: Grinding, Block size: C16. A Step Bur 12 and a Cylinder Pointed Bur 12S were used as the processing tools.

### [Processing Time]

The processing time in Tables 1 and 2 represents the duration needed to complete the processing of the first sample with a new processing tool, following the conditions outlined in the foregoing section [Processability Evaluation of Zirconia Composite Sintered Body].

If errors occurred during processing due to the processing load or some other reason, and the CEREC system MC-XL stopped working during processing, the process was resumed after replacing the tool with a new one. This procedure was repeated until one sample was fully processed, and the time required for this process was recorded as the processing time.

In Tables 1 and 2, samples that were completely unprocessable are indicated by "×" (not possible).

### [Unit Count]

The unit count in Tables 1 and 2 represents the number of samples that were processed into the shape of an anterior tooth crown without having to replace the processing tool even once during the processing conducted with a new set of processing tools under the conditions outlined in the foregoing section [Processability Evaluation of Zirconia Composite Sintered Body]. Testing involved at a maximum of 30 samples. If all 30 samples were successfully processed with a single processing tool, no further tests were conducted, and the result was recorded as "30 or more " in all such instances.

If errors occurred during processing due to the processing load or some other reason, and the MC-XL stopped working before finishing the first sample, the process was resumed after replacing the tool with a new one. This procedure was repeated until one sample was fully processed, and the reciprocal of the number of tools used was recorded as a unit count. For example, the representation "0.2" indicates that five processing tools were used to process one sample into the shape of an anterior tooth crown. The results are presented in Tables 1 and 2 below.

**[Table 1]**

| | Layer | Raw materials | | | Manufacturing conditions | | Zirconia composite sintered body | | | | | | | | |
|---|---|---|---|---|---|---|---|---|---|---|---|---|---|---|---|
| | | | | | | | Content of components | | | | | | | A/B | Aesthetic evaluation |
| | | ZrO2, stabilizer (Y2O3), Nb2O5 or Ta205 | Capping agent | Optional component | Highest sintering temp. [°C] | HIP temp. [°C] | Unit [mol%] | | | | | Elements or ions derived from capping agent [mol%] | Optional component (Ti02 or Al2O3) [mass%] | | |
| | | | | | | | (1) Zr02 and Hf02 | (2) Y203 (A) | (3) Nb2O5 (B) | (4) Ta205 (B) | (1) to (4) in total | | | | |
| Ex. 1 | First layer | ZrO2, Y2O3, Nb2O5 | NaOH | TiO2 | 1550 | 1450 | 90.13 | 5.25 | 4.62 | 0.00 | 100.00 | 0.18 | 3.00 | 1.13 | ○ |
| | Second layer | ZrO2, Y2O3, Nb2O5 | NaOH | Ti02 | | | 90.55 | 5.27 | 4.18 | 0.00 | 100.00 | 0.23 | 3.00 | 1.26 | |
| | Third layer | ZrO2, Y2O3, Nb2O5 | NaOH | Ti02 | | | 90.97 | 5.29 | 3.73 | 0.00 | 100.00 | 0.28 | 3.00 | 1.42 | |
| | Fourth layer | ZrO2, Y2O3, Nb2O5 | NaOH | Ti02 | | | 91.40 | 5.32 | 3.28 | 0.00 | 100.00 | 0.33 | 3.00 | 1.62 | |
| Ex. 2 | First layer | ZrO2, Y2O3, Nb2O5 | NaOH | TiO2 | 1550 | 1450 | 90.13 | 5.25 | 4.62 | 0.00 | 100.00 | 0.18 | 3.00 | 1.13 | ○ |
| | Second layer | ZrO2, Y2O3, Nb2O5 | NaOH | Ti02 | | | 90.55 | 5.27 | 4.18 | 0.00 | 100.00 | 0.18 | 3.00 | 1.26 | |
| | Third layer | ZrO2, Y2O3, Nb2O5 | NaOH | Ti02 | | | 90.97 | 5.29 | 3.73 | 0.00 | 100.00 | 0.18 | 3.00 | 1.42 | |
| | Fourth layer | ZrO2, Y2O3, Nb2O5 | NaOH | Ti02 | | | 91.40 | 5.32 | 3.28 | 0.00 | 100.00 | 0.18 | 3.00 | 1.62 | |
| Ex. 3 | First layer | ZrO2, Y2O3, Nb2O5 | NaOH | TiO2 | 1550 | 1450 | 90.13 | 5.25 | 4.62 | 0.00 | 100.00 | 0.18 | 3.00 | 1.13 | ○ |
| | Second layer | ZrO2, Y2O3, Nb2O5 | NaOH | Ti02 | | | 90.01 | 5.49 | 4.50 | 0.00 | 100.00 | 0.18 | 3.00 | 1.22 | |
| | Third layer | ZrO2, Y2O3, Nb2O5 | NaOH | Ti02 | | | 89.78 | 5.73 | 4.49 | 0.00 | 100.00 | 0.18 | 3.00 | 1.28 | |
| | Fourth layer | ZrO2, Y2O3, Nb2O5 | NaOH | Ti02 | | | 89.55 | 5.97 | 4.48 | 0.00 | 100.00 | 0.18 | 3.00 | 1.33 | |
| Ex. 4 | First layer | ZrO2, Y2O3, Nb2O5 | NaOH | TiO2 | 1550 | 1450 | 90.13 | 5.25 | 4.62 | 0.00 | 100.00 | 0.18 | 3.00 | 1.13 | ○ |
| | Second layer | ZrO2, Y2O3, Nb2O5 | NaOH | Ti02 | | | 90.01 | 5.49 | 4.50 | 0.00 | 100.00 | 0.23 | 3.00 | 1.22 | |
| | Third layer | ZrO2, Y2O3, Nb2O5 | NaOH | Ti02 | | | 89.78 | 5.73 | 4.49 | 0.00 | 100.00 | 0.28 | 3.00 | 1.28 | |
| | Fourth layer | ZrO2, Y203, Nb2O5 | NaOH | Ti02 | | | 89.55 | 5.97 | 4.48 | 0.00 | 100.00 | 0.33 | 3.00 | 1.33 | |
| Ex. 5 | First layer | ZrO2, Y2O3, Nb2O5 | KOH | TiO2 | 1550 | 1450 | 90.13 | 5.25 | 4.62 | 0.00 | 100.00 | 0.15 | 3.00 | 1.13 | ○ |
| | Second layer | ZrO2, Y2O3, Nb2O5 | KOH | TiO2 | | | 90.55 | 5.27 | 4.18 | 0.00 | 100.00 | 0.18 | 3.00 | 1.26 | |
| | Third layer | ZrO2, Y2O3, Nb2O5 | KOH | TiO2 | | | 90.97 | 5.29 | 3.73 | 0.00 | 100.00 | 0.21 | 3.00 | 1.42 | |
| | Fourth layer | ZrO2, Y2O3, Nb2O5 | KOH | TiO2 | | | 91.40 | 5.32 | 3.28 | 0.00 | 100.00 | 0.24 | 3.00 | 1.62 | |
| Ex. 6 | First layer | ZrO2, Y2O3, Nb2O5 | KOH | TiO2 | 1550 | 1450 | 90.13 | 5.25 | 4.62 | 0.00 | 100.00 | 0.15 | 3.00 | 1.13 | ○ |
| | Second layer | ZrO2, Y2O3, Nb2O5 | KOH | TiO2 | | | 90.55 | 5.27 | 4.18 | 0.00 | 100.00 | 0.15 | 3.00 | 1.26 | |
| | Third layer | ZrO2, Y2O3, Nb2O5 | KOH | TiO2 | | | 90.97 | 5.29 | 3.73 | 0.00 | 100.00 | 0.15 | 3.00 | 1.42 | |
| | Fourth layer | ZrO2, Y2O3, Nb2O5 | KOH | TiO2 | | | 91.40 | 5.32 | 3.28 | 0.00 | 100.00 | 0.15 | 3.00 | 1.62 | |

**[Table 1] Continued**

| | Layer | Raw materials | | | Manufacturing conditions | | Zirconia composite sintered body | | | | |
|---|---|---|---|---|---|---|---|---|---|---|---|
| | | | | | | | Properties | | | | |
| | | ZrO2, stabilizer (Y2O3), Nb2O5 or Ta2O5 | Capping agent | Optional component | Highest sintering temp. [°C] | HIP temp. [°C] | Average crystal grain size [µm] | Machinability | | Translucency ΔL*(W-B) | Biaxial flexural strength [MPa] |
| | | | | | | | | Processing time | Unit count | | |
| Ex. 1 | First layer | ZrO2, Y2O3, Nb2O5 | NaOH | TiO2 | 1550 | 1450 | 2.0 | 19m 43s | 30 or more | 15.5 | 611 |
| | Second layer | ZrO2, Y2O3, Nb2O5 | NaOH | TiO2 | | | 1.7 | | | 14.5 | 603 |
| | Third layer | ZrO2, Y2O3, Nb2O5 | NaOH | TiO2 | | | 1.6 | | | 14.1 | 601 |
| | Fourth layer | ZrO2, Y2O3, Nb2O5 | NaOH | TiO2 | | | 1.0 | | | 12.8 | 613 |
| Ex. 2 | First layer | ZrO2, Y2O3, Nb2O5 | NaOH | TiO2 | 1550 | 1450 | 2.0 | 24m 5s | 30 or more | 15.5 | 605 |
| | Second layer | ZrO2, Y2O3, Nb2O5 | NaOH | TiO2 | | | 1.8 | | | 14.8 | 619 |
| | Third layer | ZrO2, Y2O3, Nb2O5 | NaOH | TiO2 | | | 1.4 | | | 14.3 | 613 |
| | Fourth layer | ZrO2, Y2O3, Nb2O5 | NaOH | TiO2 | | | 0.8 | | | 13.1 | 619 |
| Ex. 3 | First layer | ZrO2, Y2O3, Nb2O5 | NaOH | TiO2 | 1550 | 1450 | 2.0 | 23m 56s | 30 or more | 15.5 | 610 |
| | Second layer | ZrO2, Y2O3, Nb2O5 | NaOH | TiO2 | | | 1.8 | | | 14.7 | 620 |
| | Third layer | ZrO2, Y2O3, Nb2O5 | NaOH | TiO2 | | | 1.3 | | | 14.1 | 610 |
| | Fourth layer | ZrO2, Y2O3, Nb2O5 | NaOH | TiO2 | | | 1.0 | | | 12.8 | 613 |
| Ex. 4 | First layer | ZrO2, Y203, Nb2O5 | NaOH | TiO2 | 1550 | 1450 | 2.0 | 20m 07s | 30 or more | 15.5 | 615 |
| | Second layer | ZrO2, Y2O3, Nb2O5 | NaOH | TiO2 | | | 1.8 | | | 14.6 | 608 |
| | Third layer | ZrO2, Y2O3, Nb2O5 | NaOH | TiO2 | | | 1.3 | | | 14.2 | 618 |
| | Fourth layer | ZrO2, Y2O3, Nb2O5 | NaOH | TiO2 | | | 1.0 | | | 13.0 | 602 |
| Ex. 5 | First layer | ZrO2, Y2O3, Nb2O5 | KOH | TiO2 | 1550 | 1450 | 2.0 | 20m 17s | 30 or more | 15.3 | 621 |
| | Second layer | ZrO2, Y2O3, Nb2O5 | KOH | TiO2 | | | 1.7 | | | 14.6 | 605 |
| | Third layer | ZrO2, Y2O3, Nb2O5 | KOH | TiO2 | | | 1.5 | | | 14.0 | 610 |
| | Fourth layer | ZrO2, Y2O3, Nb2O5 | KOH | TiO2 | | | 1.1 | | | 13.2 | 590 |
| Ex. 6 | First layer | ZrO2, Y2O3, Nb2O5 | KOH | TiO2 | 1550 | 1450 | 2.1 | 23m 14s | 30 or more | 15.3 | 614 |
| | Second layer | ZrO2, Y2O3, Nb2O5 | KOH | TiO2 | | | 1.8 | | | 14.7 | 613 |
| | Third layer | ZrO2, Y2O3, Nb2O5 | KOH | TiO2 | | | 1.4 | | | 14.2 | 603 |
| | Fourth layer | ZrO2, Y2O3, Nb2O5 | KOH | TiO2 | | | 0.9 | | | 13.3 | 613 |

**[Table 2]**

| | Layer | Raw materials | | | Manufacturing conditions | | Zirconia composite sintered body | | | | | | | | |
|---|---|---|---|---|---|---|---|---|---|---|---|---|---|---|---|
| | | | | | | | Content of components | | | | | | | A/B | Aesthetic evaluation |
| | | Zr02, stabilizer (Y203), Nb205 or Ta205 | Capping agent | Optional component | Highest sintering temp. [°C] | HIP temp. [°C] | Unit [mol%] | | | | | Elements or ions derived from capping agent [mol%] | Optional component (Ti02 or Al2O3) [mass%] | | |
| | | | | | | | (1) Zr02 and Hf02 | (2) Y203 (A) | (3) Nb2O5 (B) | (4) Ta205 (B) | (1) to (4) in total | | | | |
| Ex. 7 | First layer | ZrO2, Y2O3, Nb2O5 | NaOH | TiO2 | 1550 | 1450 | 90.34 | 5.26 | 4.40 | 0.00 | 100.00 | 0.18 | 3.00 | 1.19 | ○ |
| | Second layer | Zr02, Y203, Nb205 | NaOH | Ti02 | | | 90.55 | 5.27 | 4.18 | 0.00 | 100.00 | 0.18 | 3.00 | 1.26 | |
| | Third layer | Zr02, Y203, Nb205 | NaOH | Ti02 | | | 90.76 | 5.28 | 3.96 | 0.00 | 100.00 | 0.18 | 3.00 | 1.33 | |
| | Fourth layer | Zr02, Y203, Nb205 | NaOH | Ti02 | | | 90.97 | 5.29 | 3.73 | 0.00 | 100.00 | 0.18 | 3.00 | 1.42 | |
| Ex. 8 | First layer | Zr02, Y203, Nb205 | NaOH | TiO2 | 1550 | 1450 | 89.92 | 5.23 | 4.85 | 0.00 | 100.00 | 0.18 | 3.00 | 1.08 | ○ |
| | Second layer | Zr02, Y203, Nb205 | NaOH | Ti02 | | | 90.13 | 5.25 | 4.62 | 0.00 | 100.00 | 0.18 | 3.00 | 1.13 | |
| | Third layer | Zr02, Y203, Nb205 | NaOH | Ti02 | | | 90.34 | 5.26 | 4.40 | 0.00 | 100.00 | 0.18 | 3.00 | 1.19 | |
| | Fourth layer | Zr02, Y203, Nb205 | NaOH | Ti02 | | | 90.55 | 5.27 | 4.18 | 0.00 | 100.00 | 0.18 | 3.00 | 1.26 | |
| Ex. 9 | First layer | Zr02, Y203, Nb205 | NaOH | TiO2 | 1550 | 1450 | 90.76 | 5.28 | 3.96 | 0.00 | 100.00 | 0.18 | 3.00 | 1.33 | ○ |
| | Second layer | Zr02, Y203, Nb205 | NaOH | Ti02 | | | 90.97 | 5.29 | 3.73 | 0.00 | 100.00 | 0.18 | 3.00 | 1.42 | |
| | Third layer | Zr02, Y203, Nb205 | NaOH | Ti02 | | | 91.19 | 5.31 | 3.51 | 0.00 | 100.00 | 0.18 | 3.00 | 1.51 | |
| | Fourth layer | Zr02, Y203, Nb205 | NaOH | Ti02 | | | 91.40 | 5.32 | 3.28 | 0.00 | 100.00 | 0.18 | 3.00 | 1.62 | |
| Ex. 10 | First layer | Zr02, Y203, Nb205 | NaOH | TiO2 | 1550 | 1450 | 90.34 | 5.26 | 4.40 | 0.00 | 100.00 | 0.18 | 3.00 | 1.19 | ○ |
| | Second layer | Zr02, Y203, Nb205 | NaOH | Ti02 | | | 90.76 | 5.28 | 3.96 | 0.00 | 100.00 | 0.18 | 3.00 | 1.33 | |
| | Third layer | Zr02, Y203, Nb205 | NaOH | Ti02 | | | 91.19 | 5.31 | 3.51 | 0.00 | 100.00 | 0.18 | 3.00 | 1.51 | |
| Ex. 11 | First layer | Zr02, Y203, Nb205 | NaOH | TiO2 | 1550 | 1450 | 89.71 | 5.22 | 5.07 | 0.00 | 100.00 | 0.18 | 3.00 | 1.03 | ○ |
| | Second layer | Zr02, Y203, Nb205 | NaOH | Ti02 | | | 89.92 | 5.23 | 4.85 | 0.00 | 100.00 | 0.18 | 3.00 | 1.08 | |
| | Third layer | Zr02, Y203, Nb205 | NaOH | Ti02 | | | 90.34 | 5.26 | 4.40 | 0.00 | 100.00 | 0.18 | 3.00 | 1.19 | |
| | Fourth layer | Zr02, Y203, Nb205 | NaOH | Ti02 | | | 90.76 | 5.28 | 3.96 | 0.00 | 100.00 | 0.18 | 3.00 | 1.33 | |
| | Fifth layer | Zr02, Y203, Nb205 | NaOH | Ti02 | | | 91.19 | 5.31 | 3.51 | 0.00 | 100.00 | 0.18 | 3.00 | 1.51 | |
| Com. Ex. 1 | First layer | Zr02, Y203 | - | - | 1550 | - | 93.00 | 7.00 | 0.00 | 0.00 | 100.00 | - | - | - | ○ |
| | Second layer | Zr02, Y203 | - | - | | | 95.00 | 5.00 | 0.00 | 0.00 | 100.00 | - | - | - | |
| | Third layer | Zr02, Y203 | - | - | | | 97.00 | 3.00 | 0.00 | 0.00 | 100.00 | - | - | - | |
| Com. Ex. 2 | Single layer | Zr02, Y203, Nb205 | NaOH | TiO2 | 1550 | 1450 | 90.30 | 5.30 | 4.40 | 0.00 | 100.00 | 0.18 | 3.00 | 1.2 | × |

**[Table 2] Continued**

| | Layer | Raw materials | | | Manufacturing conditions | | Zirconia composite sintered body | | | | |
|---|---|---|---|---|---|---|---|---|---|---|---|
| | | | | | | | Properties | | | | |
| | | ZrO2, stabilizer (Y2O3), Nb2O5 or Ta2O5 | Capping agent | Optional component | Highest sintering temp. [°C] | HIP temp. [°C] | Average crystal grain size [µm] | Machinability | | Translucency ΔL*(W-B) | Biaxial flexural strength [MPa] |
| | | | | | | | | Processing time | Unit count | | |
| Ex. 7 | First layer | ZrO2, Y2O3, Nb2O5 | NaOH | TiO2 | 1550 | 1450 | 1.9 | 23m 40s | 30 or more | 15.1 | 618 |
| | Second layer | ZrO2, Y2O3, Nb2O5 | NaOH | TiO2 | | | 1.7 | | | 14.5 | 600 |
| | Third layer | ZrO2, Y2O3, Nb2O5 | NaOH | TiO2 | | | 1.6 | | | 14.3 | 623 |
| | Fourth layer | ZrO2, Y2O3, Nb2O5 | NaOH | TiO2 | | | 1.5 | | | 14.0 | 613 |
| Ex. 8 | First layer | ZrO2, Y2O3, Nb2O5 | NaOH | TiO2 | 1550 | 1450 | 2.1 | 22m 26s | 30 or more | 15.8 | 554 |
| | Second layer | ZrO2, Y2O3, Nb2O5 | NaOH | TiO2 | | | 2.0 | | | 15.5 | 602 |
| | Third layer | ZrO2, Y2O3, Nb2O5 | NaOH | TiO2 | | | 1.9 | | | 15.0 | 623 |
| | Fourth layer | ZrO2, Y2O3, Nb2O5 | NaOH | TiO2 | | | 1.7 | | | 14.5 | 619 |
| Ex. 9 | First layer | ZrO2, Y2O3, Nb2O5 | NaOH | TiO2 | 1550 | 1450 | 1.6 | 26m 46s | 14 | 14.3 | 611 |
| | Second layer | ZrO2, Y2O3, Nb2O5 | NaOH | TiO2 | | | 1.5 | | | 14.1 | 620 |
| | Third layer | ZrO2, Y2O3, Nb2O5 | NaOH | TiO2 | | | 1.2 | | | 13.5 | 610 |
| | Fourth layer | ZrO2, Y2O3, Nb2O5 | NaOH | TiO2 | | | 0.9 | | | 12.9 | 615 |
| Ex. 10 | First layer | ZrO2, Y2O3, Nb2O5 | NaOH | TiO2 | 1550 | 1450 | 1.9 | 23m 43s | 30 or more | 15.1 | 598 |
| | Second layer | ZrO2, Y2O3, Nb2O5 | NaOH | TiO2 | | | 1.6 | | | 14.4 | 603 |
| | Third layer | ZrO2, Y2O3, Nb2O5 | NaOH | TiO2 | | | 1.2 | | | 13.6 | 627 |
| Ex. 11 | First layer | ZrO2, Y2O3, Nb2O5 | NaOH | TiO2 | 1550 | 1450 | 2.3 | 22m 48s | 30 or more | 16.0 | 512 |
| | Second layer | ZrO2, Y2O3, Nb2O5 | NaOH | TiO2 | | | 2.1 | | | 15.7 | 567 |
| | Third layer | ZrO2, Y2O3, Nb2O5 | NaOH | TiO2 | | | 1.9 | | | 15.0 | 611 |
| | Fourth layer | ZrO2, Y2O3, Nb2O5 | NaOH | TiO2 | | | 1.6 | | | 14.3 | 603 |
| | Fifth layer | ZrO2, Y2O3, Nb2O5 | NaOH | TiO2 | | | 1.2 | | | 13.5 | 608 |
| Com. Ex. 1 | First layer | ZrO2, Y2O3 | - | - | 1550 | - | 3.1 | × | 0 | 17.5 | 480 |
| | Second layer | ZrO2, Y2O3 | - | - | | | 1.5 | | | 15.2 | 602 |
| | Third layer | ZrO2, Y2O3 | - | - | | | 0.5 | | | 12.0 | 931 |
| Com. Ex. 2 | Single layer | ZrO2, Y2O3, Nb2O5 | NaOH | TiO2 | 1550 | 1450 | 2.0 | 19m 43s | 30 or more | 15.5 | 604 |

These results confirmed that the zirconia composite sintered bodies of the present invention can exhibit excellent aesthetic properties with sufficient gradational translucency while possessing strength suited for dental use and superior machinability in the sintered state.

In Examples 1 to 11, it was possible to reduce wear on processing tools, greatly increasing the number of dental prostheses that can be continuously produced with a single processing tool.

In contrast, in Comparative Example 1, containing no Nb₂O₅ or Ta₂O₅, machining failed completely in the sintered state.

Comparative Example 2, which does not include a layered structure with a plurality of layers differing in the content of at least one component from among a stabilizer, Nb₂O₅, and Ta₂O₅, was inferior in terms of aesthetics because of the high overall translucency with excessive transparency in a portion corresponding to the cervical region.

### INDUSTRIAL APPLICABILITY

A zirconia composite sintered body of the present invention can exhibit excellent aesthetic properties while possessing strength suited for dental use and superior machinability in the sintered state. This makes a zirconia composite sintered body of the present invention particularly useful as a dental prosthesis or other dental materials intended for dental treatment.

## Claims

1. A zirconia composite sintered body comprising zirconia, a stabilizer capable of preventing a phase transformation of zirconia, and at least one of Nb₂O₅ or Ta₂O₅, and having a plurality of layers that differ in the content of at least one component from among the stabilizer, Nb₂O₅, and Ta₂O₅ relative to the total moles of zirconia, the stabilizer, Nb₂O₅, and Ta₂O₅.

2. The zirconia composite sintered body according to claim 1, wherein the plurality of layers are layers that differ in the content of Nb₂O₅ or Ta₂O₅.

3. The zirconia composite sintered body according to claim 2, wherein the content of Nb₂O₅ or Ta₂O₅ relative to the total moles of zirconia, the stabilizer, Nb₂O₅, and Ta₂O₅ shows an unchanging pattern of increase or decrease from one end to the other end of the zirconia composite sintered body on a straight line extending along a first direction from one end to the other end of the zirconia composite sintered body.

4. The zirconia composite sintered body according to claim 3, wherein the stabilizer is yttria.

5. The zirconia composite sintered body according to claim 4, wherein the content of Nb₂O₅ or Ta₂O₅ is 2 mol% or more and 12 mol% or less in a layer including said one end, and the content of Nb₂O₅ or Ta₂O₅ is 1 mol% or more and 10 mol% or less in a layer including said other end, relative to the total moles of zirconia, yttria, Nb₂O₅, and Ta₂O₅.

6. The zirconia composite sintered body according to claim 1, wherein the plurality of layers are layers that differ in the content of the stabilizer.

7. The zirconia composite sintered body according to claim 6, wherein the content of the stabilizer relative to the total moles of zirconia, the stabilizer, Nb₂O₅, and Ta₂O₅ shows an unchanging pattern of increase or decrease from one end to the other end of the zirconia composite sintered body on a straight line extending along a first direction from one end to the other end of the zirconia composite sintered body.

8. The zirconia composite sintered body according to claim 7, wherein the stabilizer is yttria.

9. The zirconia composite sintered body according to claim 8, wherein the content of yttria is 1 mol% or more and 8 mol% or less in a layer including said one end, and the content of yttria is 2 mol% or more and 9 mol% or less in a layer including said other end, relative to the total moles of zirconia, yttria, Nb₂O₅, and Ta₂O₅.

10. The zirconia composite sintered body according to claim 1 or 2, wherein at least one of the layers in said plurality of layers further comprises an element or ion derived from a capping agent.

11. The zirconia composite sintered body according to claim 10, wherein the content of the element or ion derived from a capping agent is more than 0 mol% and 5 mol% or less relative to total 100 mol% of zirconia, the stabilizer, Nb₂O₅, and Ta₂O₅.

12. The zirconia composite sintered body according to claim 10, wherein the element or ion derived from a capping agent belongs to periods 2 to 7 of the periodic table, and is an element or an ion thereof having a first ionization energy lower than that of a group 18 element of the same period, and/or an element or an ion thereof having a high electron affinity.

13. The zirconia composite sintered body according to claim 10, wherein the element or ion derived from a capping agent comprises at least one element or an ion thereof selected from the group consisting of Cu, Ag, Li, Na, K, Rb, Cs, Fr, At, I, Br, Cl, and F.

14. The zirconia composite sintered body according to claim 10, wherein the element or ion derived from a capping agent comprises at least one element or an ion thereof selected from the group consisting of Li, Na, K, Rb, Cs, and Fr.

15. The zirconia composite sintered body according to claim 1 or 2, wherein at least one of the layers in said plurality of layers satisfies a ratio A/B of 0.9 or more and 3 or less, where A is the content of the stabilizer in mol%, and B is the combined content of Nb₂O₅ and Ta₂O₅ in mol%.

16. The zirconia composite sintered body according to claim 1 or 2, wherein at least one of the layers in said plurality of layers further comprises a zirconia enhancer, and the content of the zirconia enhancer is more than 0 mass% and 6.0 mass% or less relative to total 100 mass% of zirconia, the stabilizer, and Nb₂O₅ and Ta₂O₅.

17. The zirconia composite sintered body according to claim 16, wherein the zirconia enhancer comprises TiO₂ and/or Al₂O₃.

18. The zirconia composite sintered body according to claim 1 or 2, which has an average crystal grain size of 0.5 to 5.0 µm.
